(19) Europäisches Patentamt
European Patent Office
Office européen des brevets

(11) Veröffentlichungsnummer: **0 107 116**
**B1**

(12) # EUROPÄISCHE PATENTSCHRIFT

(45) Veröffentlichungstag der Patentschrift: **27.08.86**

(21) Anmeldenummer: **83109854.6**

(22) Anmeldetag: **03.10.83**

(51) Int. Cl.⁴: **C 07 C 121/75**, C 07 C 121/60, C 07 C 121/00, C 09 K 19/14, C 09 K 19/30, C 09 K 19/42, C 09 K 19/44, C 09 K 19/46, C 09 K 19/48

(54) **Dicyanobenzole.**

(30) Priorität: **21.10.82 CH 6124/82**
**28.07.83 CH 4144/83**

(43) Veröffentlichungstag der Anmeldung: **02.05.84 Patentblatt 84/18**

(45) Bekanntmachung des Hinweises auf die Patenterteilung: **27.08.86 Patentblatt 86/35**

(84) Benannte Vertragsstaaten: **CH DE GB LI**

(56) Entgegenhaltungen:
**EP-A-0 023 728**
**EP-A-0 084 194**
**EP-A-0 085 995**
**EP-A-0 087 963**

**MOLECULAR CRYSTALS AND LIQUID CRYSTALS & LETTERS, Band 82, Nr. 10, 1983, Seiten 339-344, Gordon and Breach, Science Publishers, Inc., US**

(73) Patentinhaber: **F. HOFFMANN-LA ROCHE & CO. Aktiengesellschaft, CH-4002 Basel (CH)**

(72) Erfinder: **Boller, Arthur, Dr., Benkenstrasse 65, CH-4102 Binningen (CH)**
Erfinder: **Petrzilka, Martin, Dr., Violaweg 74, CH-4303 Kaiseraugst (CH)**
Erfinder: **Schadt, Martin, Dr., Liestalerstrasse 77, CH-4411 Seltisberg (CH)**

(74) Vertreter: **Cottong, Norbert A. et al, Grenzacherstrasse 124 Postfach 3255, CH-4002 Basel (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die vorliegende Erfindung betrifft neue flüssigkristalline Gemische gemäss Anspruch 12 enthaltend Dicyanobenzole der allgemeinen Formel

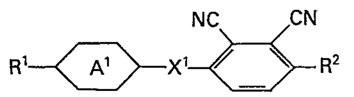

I

worin $R^1$ und $R^2$ geradkettiges $C_1$-$C_{12}$-Alkyl oder an einem aromatischen Ring auch geradkettiges $C_1$-$C_{12}$-Alkoxy bedeuten, oder einer der Reste $R^1$ und $R^2$ auch eine Gruppe der allgemeinen Formel

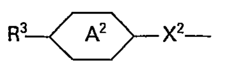

II

bedeutet; $X^1$ und $X^2$ einfache Kovalenzbindungen oder eine dieser Gruppen auch eine Äthylengruppe $-CH_2CH_2-$ bezeichnen; die Ringe $A^1$ und $A^2$ 1,4-Phenylen oder, sofern $X^1$ oder $X^2$ eine Äthylengruppe $-CH_2CH_2-$ bezeichnet, auch trans-1,4-Cyclohexylen darstellen; und $R^3$ geradkettiges $C_1$-$C_{12}$-Alkyl oder an einem aromatischen Ring $A^2$ auch geradkettiges $C_1$-$C_{12}$-Alkoxy bedeutet, und flüssigkristalline Gemische gemäss Ansprüchen 11 und 13 enthaltend Verbindungen der Formel I mit einer Äthylengruppe $X^1$ oder $X^2$.

Die Erfindung betrifft ferner die Herstellung der Verbindungen der in Anspruch 11 definierten Formel I nach dem Verfahren von Anspruch 14 sowie die neuen Dicyanobenzole gemäss Anspruch 1 und deren Verwendung für elektro-optische Zwecke.

Der obige Ausdruck «geradkettiges $C_1$-$C_{12}$-Alkyl» umfasst Methyl, Äthyl, Propyl, Butyl, Pentyl, Hexyl, Heptyl, Octyl, Nonyl, Decyl, Undecyl und Dodecyl, und der Ausdruck «geradkettiges $C_1$-$C_{12}$-Alkoxy» umfasst Methoxy, Äthoxy, Propyloxy, Butyloxy, Pentyloxy, Hexyloxy, Heptyloxy, Octyloxy, Nonyloxy, Decyloxy, Undecyloxy und Dodecyloxy.

Soweit nichts Gegenteiliges erwähnt wird, bedeuten die Ausdrücke «dielektrische Anisotropie» bzw. «Anisotropie der Dielektrizitätskonstante» im Rahmen der vorliegenden Erfindung die niederfrequente («statische») dielektrische Anisotropie.

Nematische und cholesterische Flüssigkristalle mit negativer Anisotropie der Dielektrizitätskonstante ($\Delta\varepsilon = \varepsilon_{\parallel} - \varepsilon_{\perp} < 0$, wobei $\varepsilon_{\parallel}$ die Dielektrizitätskonstante entlang der Moleküllängsachse und $\varepsilon_{\perp}$ die Dielektrizitätskonstante senkrecht dazu bedeuten) werden in einem elektrischen Feld mit ihren Moleküllängsachsen senkrecht zur Feldrichtung ausgerichtet. Dieser Effekt ist bekannt und wird zur Steuerung der optischen Transparenz in verschiedenen Flüssigkristallanzeigen ausgenützt, so z. B. in Flüssigkristallzellen vom Lichtstreuungstyp (dynamische Streuung), vom sogenannten DAP-Typ (Deformation aufgerichteter Phasen) oder vom Gast/Wirt-Typ (guest host interaction).

Bei der «Guest/Host-Zelle» handelt es sich im wesentlichen um einen Kondensator, wobei mindestens eine Elektrode lichtdurchlässig ist und das Dielektrikum von einem nematischen oder cholesterischen Flüssigkristall gebildet wird, welcher einen oder mehrere dichroitische Farbstoffe enthält. Da die verwendbaren Farbstoffe meist positiven Dichroismus aufweisen, d. h. das Übergangsmoment der Absorption von sichtbarem Licht annähernd in Richtung der Moleküllängsachse liegt, entspricht die Ausrichtung des Flüssigkristalls mit den Moleküllängsachsen parallel zur Plattenoberfläche im allgemeinen dem farbigen Zustand und die homöotrope Ausrichtung (Moleküllängsachsen senkrecht zur Plattenoberfläche) dem farblosen Zustand der Zelle. Bei Verwendung eines Flüssigkristalls mit positiver dielektrischer Anisotropie wird dessen homogene Orientierung (welche durch Behandlung der Plattenoberfläche erreicht wird) durch Anlegen einer Spannung homöotrop ausgerichtet, d. h. die Zelle wird von «farbig» auf «farblos» geschaltet. Auf diese Weise werden farblose Zeichen auf farbigem Untergrund angezeigt. Demgegenüber wird bei Verwendung eines Flüssigkristalls mit negativer dielektrischer Anisotropie dessen homöotrope Orientierung (durch Behandlung der Plattenoberfläche) durch Anlegen einer Spannung parallel zu den Elektrodenoberflächen ausgerichtet, wodurch die Anzeige farbiger Bildelemente auf farblosem Untergrund ermöglicht wird.

Ferner wurde zur Verbesserung des Multiplexverhältnisses bei der Multiplex-Ansteuerung von Flüssigkristallanzeigen, insbesondere von Drehzellen und Guest/Host-Zellen, eine Zwei-Frequenz-Matrix-Adressierung vorgeschlagen (z. B. Deutsche Offenlegungsschriften 2856134 und 2907940). Hierbei wird der Umstand ausgenützt, dass die dielektrische Anisotropie von Flüssigkristallen, welche beim Anlagen einer niederfrequenten Spannung eine positive Anisotropie der Dielektrizitätskonstante besitzen, bei hohen Frequenzen negativ wird. Um die kapazitiven Verluste klein zu halten, sollte die «Cross-over-Frequenz» $f_c$ (dielektrische Relaxationsfrequenz, bei welcher $\varepsilon_{\parallel} = \varepsilon_{\perp}$ wird) derartiger Flüssigkristalle möglichst niedrig sein und nicht über etwa 20 kHz liegen. Ferner sollte der absolute Betrag der dielektrischen Anisotropie sowohl unterhalb als auch oberhalb der Cross-over-Frequenz möglichst gross sein. Es hat sich jedoch gezeigt, dass die Substanzen, welche für das Zwei-Frequenz-Verfahren besonders geeignet sind, bei Frequenzen oberhalb der Cross-over-Frequenz im allgemeinen eine kleinere absolute dielektrische Anisotropie aufweisen als unterhalb der Cross-over-Frequenz. Dieser Nachteil könnte behoben werden durch Zusatz von Verbindungen mit negativer dielektrischer Anisotropie und geeignetem Relaxationsverhalten.

Es sind bisher bereits eine Reihe von flüssigkristallinen Verbindungen mit schwach negativer dielektrischer Anisotropie synthetisiert worden. Hingegen sind noch relativ wenig Flüssigkristallkomponenten mit grosser negativer Anisotropie der Dielektrizitätskonstante bekannt. Zudem weisen letztere im allgemeinen Nachteile auf, wie z. B. schlechte Löslichkeit in Mischungen, hohe Viskosität, hohe Schmelzpunkte, starke smektische Tendenzen und chemische Instabilität. Es besteht daher ein Bedarf an weiteren Verbindungen mit negativer dielektrischer Anisotropie, die es erlauben, die Eigenschaften von Mischungen für verschiedenste elektro-optische Anwendungen weiter zu verbessern.

Flüssigkristallkomponenten mit negativer dielektrischer Anisotropie, welche zwei oder drei über Carboxylgruppen oder Kovalenzbindungen verknüpfte Ringe und eine oder mehrere seitliche Gruppen, wie Halogen, Cyano, oder Nitrogruppen aufweisen, sind bekannt aus DE-AS 2240864, DE-OS 2613293, DE-OS 2835662, DE-OS 2836086 und EP-A-O 023728. EP-A-O 023728 beschreibt unter anderem auch Verbindungen mit einer 2,3-Dicyano-1,4-phenylengruppe beispielsweise 2,3-Dicyano-4-propylphenyl-4'-alkylcyclohexane, worin Alkyl Propyl, Pentyl oder Heptyl bedeutet. Diese 2,3-Dicyanobenzole weisen mindestens einen Cyclohexanring auf und die Ringe sind im Unterschied zu den Verbindungen der obigen Formel I über einfache Kovalenzbindungen und/oder Carboxyl- oder Oxocarbonylgruppen verknüpft.

Es wurde nun gefunden, dass die Verbindungen der obigen Formel I eine grosse negative Anisotropie der Dielektrizitätskonstante, eine gute Löslichkeit in bekannten Flüssigkristallmischungen und eine relativ niedrige Viskosität aufweisen. Sie sind farblos und besitzen eine sehr gute chemische und photochemische Stabilität. Ferner können mit den Verbindungen der obigen Formel I Mischungen hergestellt werden, welche ein verbessertes Schmelzverhalten und keine oder nur geringe smektische Tendenzen aufweisen. Sie sind somit besonders geeignet, um die Eigenschaften von Flüssigkristallmischungen mit negativer Anisotropie der Dielektrizitätskonstante bzw. von Flüssigkristallmischungen, welche für die Zwei-Frequenz-Matrix-Adressierung geeignet sind, zu verbessern. Sie können aber auch in Mischungen mit positiver dielektrischer Anisotropie verwendet werden, beispielsweise um die Schwellenspannung an die verwendete elektrooptische Zelle anzupassen.

Verbindungen der Formel I, worin $R^1$ und $R^2$ geradkettiges $C_1$-$C_{12}$-Alkyl oder an einem aromatischen Ring auch geradkettiges $C_1$-$C_2$-Alkoxy bedeuten, sind bevorzugt. Im Falle dieser bicyclischen Verbindungen bezeichnen entweder $X^1$ eine einfache Kovalenzbindung und Ring $A^1$ 1,4-Phenylen, oder $X^1$ eine Äthylengruppe und Ring $A^1$ 1,4-Phenylen, oder, vorzugsweise, $X^1$ eine Äthylengruppe und Ring $A^1$ trans-1,4-Cyclohexylen.

Grundsätzlich sind solche Verbindungen der Formel I bevorzugt, worin mindestens eine der beiden Seitenketten ($R^1$, $R^2$ bzw. $R^3$), vorzugsweise beide, geradkettiges $C_1$-$C_{12}$-Alkyl bedeutet. Besonders bevorzugt sind somit diejenigen Verbindungen der Formel I, worin $R^1$ und $R^2$ geradkettiges Alkyl bedeuten, und insbesondere diejenigen, worin zusätzlich $X^1$ eine Äthylengruppe und Ring $A^1$ trans-1,4-Cyclohexylen bezeichnen.

Ferner sind grundsätzlich diejenigen Verbindungen der Formel I bevorzugt, worin die Alkylreste 3 bis 7 Kohlenstoffatome und die Alkoxyreste 2 bis 6 Kohlenstoffatome aufweisen.

Beispiele bevorzugter Verbindungen der Formel I sind diejenigen, worin $R^1$, Ring $A^1$, $X^1$ und $R^2$ die in Tabelle 1 gegebenen Bedeutungen haben ($C_6H_4$ bezeichnet 1,4-Phenylen, $C_6H_{10}$ trans-1,4-Cyclohexylen und ein einfacher Strich (–) für $X^1$ eine einfache Kovalenzbindung), sowie die weiteren in den Synthesebeispielen genannten Verbindungen der Formel I.

Tabelle 1

| $R^1$ | $A^1$ | $X^1$ | $R^2$ |
|---|---|---|---|
| $C_3H_7$ | $C_6H_4$ | – | $C_3H_7$ |
| $C_3H_7$ | $C_6H_4$ | – | $C_5H_{11}$ |
| $C_3H_7$ | $C_6H_4$ | – | $C_7H_{15}$ |
| $C_5H_{11}$ | $C_6H_4$ | – | $C_3H_7$ |
| $C_5H_{11}$ | $C_6H_4$ | – | $C_5H_{11}$ |
| $C_5H_{11}$ | $C_6H_4$ | – | $C_7H_{15}$ |
| $C_7H_{15}$ | $C_6H_4$ | – | $C_3H_7$ |
| $C_7H_{15}$ | $C_6H_4$ | – | $C_5H_{11}$ |
| $C_4H_9O$ | $C_6H_4$ | – | $C_3H_7$ |
| $C_4H_9O$ | $C_6H_4$ | – | $C_5H_{11}$ |
| $C_4H_9O$ | $C_6H_4$ | – | $C_6H_{13}$ |
| $C_3H_7$ | $C_6H_4$ | – | $C_4H_9O$ |
| $C_5H_{11}$ | $C_6H_4$ | – | $C_4H_9O$ |
| $C_3H_7$ | $C_6H_4$ | $-CH_2CH_2-$ | $C_7H_{15}$ |
| $C_5H_{11}$ | $C_6H_4$ | $-CH_2CH_2-$ | $C_3H_7$ |
| $C_5H_{11}$ | $C_6H_4$ | $-CH_2CH_2-$ | $C_5H_{11}$ |
| $C_5H_{11}$ | $C_6H_4$ | $-CH_2CH_2-$ | $C_7H_{15}$ |
| $C_7H_{15}$ | $C_6H_4$ | $-CH_2CH_2-$ | $C_3H_7$ |
| $C_4H_9O$ | $C_6H_4$ | $-CH_2CH_2-$ | $C_3H_7$ |
| $C_4H_9O$ | $C_6H_4$ | $-CH_2CH_2-$ | $C_5H_{11}$ |
| $C_5H_{11}$ | $C_6H_4$ | $-CH_2CH_2-$ | $C_4H_9O$ |
| $C_2H_5$ | $C_6H_{10}$ | $-CH_2CH_2-$ | $C_3H_7$ |
| $C_3H_7$ | $C_6H_{10}$ | $-CH_2CH_2-$ | $C_6H_{13}$ |
| $C_3H_7$ | $C_6H_{10}$ | $-CH_2CH_2-$ | $C_7H_{15}$ |
| $C_5H_{11}$ | $C_6H_{10}$ | $-CH_2CH_2-$ | $C_3H_7$ |
| $C_5H_{11}$ | $C_6H_{10}$ | $-CH_2CH_2-$ | $C_4H_9$ |
| $C_5H_{11}$ | $C_6H_{10}$ | $-CH_2CH_2-$ | $C_5H_{11}$ |
| $C_5H_{11}$ | $C_6H_{10}$ | $-CH_2CH_2-$ | $C_7H_{15}$ |
| $C_7H_{15}$ | $C_6H_{10}$ | $-CH_2CH_2-$ | $C_3H_7$ |
| $C_5H_{11}$ | $C_6H_{10}$ | $-CH_2CH_2-$ | $C_4H_9O$ |
| $C_3H_7$ | $C_6H_4$ | – | $C_5H_{11}-C_6H_4$ |
| $C_3H_7$ | $C_6H_4$ | – | $C_7H_{15}-C_6H_4$ |
| $C_5H_{11}$ | $C_6H_4$ | – | $C_3H_7-C_6H_4$ |
| $C_5H_{11}$ | $C_6H_4$ | – | $C_5H_{11}-C_6H_4$ |
| $C_5H_{11}$ | $C_6H_4$ | – | $C_7H_{15}-C_6H_4$ |
| $C_7H_{15}$ | $C_6H_4$ | – | $C_3H_7-C_6H_4$ |
| $C_7H_{15}$ | $C_6H_4$ | – | $C_5H_{11}-C_6H_4$ |
| $C_4H_9O$ | $C_6H_4$ | – | $C_5H_{11}-C_6H_4$ |
| $C_5H_{11}$ | $C_6H_4$ | – | $C_4H_9O-C_6H_4$ |
| $C_3H_7-C_6H_4$ | $C_6H_4$ | – | $C_5H_{11}$ |
| $C_5H_{11}-C_6H_4$ | $C_6H_4$ | – | $C_3H_7$ |
| $C_5H_{11}-C_6H_4$ | $C_6H_4$ | – | $C_5H_{11}$ |
| $C_7H_{15}-CV_6H_4$ | $C_6H_4$ | – | $C_3H_7$ |
| $C_4H_9O-C_6H_4$ | $C_6H_4$ | – | $C_5H_{11}$ |
| $C_5H_{11}-C_6H_4$ | $C_6H_4$ | – | $C_4H_9O$ |
| $C_5H_{11}-C_6H_{10}-CH_2CH_2-$ | $C_6H_4$ | – | $C_3H_7$ |
| $C_5H_{11}-C_6H_{10}-CH_2CH_2-$ | $C_6H_4$ | – | $C_5H_{11}$ |
| $C_7H_{15}-C_6H_{10}-CH_2CH_2-$ | $C_6H_4$ | – | $C_3H_7$ |

Die Verbindungen der Formel I können dadurch hergestellt werden, dass man

a) zur Herstellung der Verbindungen der Formel I, worin $R^2$ geradkettiges $C_1$-$C_{12}$-Alkyl oder eine

Gruppe der Formel II bedeutet, eine Verbindung der allgmeinen Formel

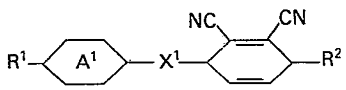

III

worin $R^2$ geradkettiges $C_1$-$C_{12}$-Alkyl oder eine Gruppe der Formel II darstellt und $R^1$, $X^1$ und Ring $A^1$ die oben gegebenen Bedeutungen haben, oxidiert, oder

b) zur Herstellung der Verbindungen der Formel I, worin $R^2$ geradkettiges $C_1$-$C_{12}$-Alkyl oder eine Gruppe der Formel II bedeutet, in einer Verbindung der allgemeinen Formel

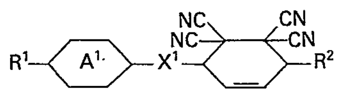

IV

worin $R^2$ geradkettiges $C_1$-$C_{12}$-Alkyl oder eine Gruppe der Formel II darstellt und $R^1$, $X^1$ und Ring $A^1$ die oben gegebenen Bedeutungen haben, Cyanwasserstoff abspaltet, oder

c) zur Herstellung der Verbindungen der Formel I, worin $R^2$ geradkettiges $C_1$-$C_{12}$-Alkoxy bedeutet, eine Verbindung der allgemeinen Formel

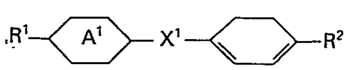

V

worin $R^2$ geradkettiges $C_1$-$C_{12}$-Alkoxy darstellt und $R^1$, $X^1$ und Ring $A^1$ die oben gegebenen Bedeutungen haben, mit Dicyanoacetylen umsetzt und anschliessend Äthylen abspaltet.

Diese Umsetzungen können nach an sich bekannten Methoden durchgeführt werden. Die Oxidation einer Verbindung der Formel III erfolgt vorzugsweise mit 2,3-Dichloro-5,6-dicyano-p-benzochinon in Dioxan oder durch katalytische Dehydrierung (bevorzugt in Gegenwart eines Palladiumkatalysators). Die Abspaltung von Cyanwasserstoff in den Verbindungen der Formel IV wird vorzugsweise mit Cäsiumfluorid in Dimethylformamid durchgeführt. Die Umsetzung einer Verbindung der Formel V mit Dicyanoacetylen kann nach den Methoden der Diels-Alder-Reaktion, vorzugsweise in einem Äther (z.B. Tetrahydrofuran), erfolgen; die anschliessende Abspaltung von Äthylen aus dem erhaltenen Diels-Alder-Primäraddukt erfolgt vorzugsweise durch Erhitzen.

Die Verbindungen der obigen Formeln sind neu.

Die Verbindungen der Formel III können beispielsweise dadurch erhalten werden, dass man einen Aldehyd der allgemeinen Formel

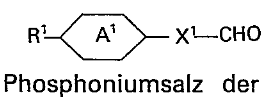

VI

mit einem Phosphoniumsalz der allgemeinen Formel

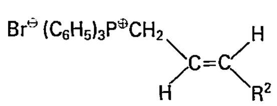

VII

worin $R^1$, $R^2$, $X^1$ und Ring $A^1$ die in Formel III gegebenen Bedeutungen haben, in Diäthyläther in Gegenwart von Butyllithium umsetzt und das erhaltene Dien durch Diels-Alder-Reaktion mit Dicyanoacetylen in Tetrahydrofuran in eine Verbindung der Formel III überführt. Die Verbindungen der Formel VII und die Verbindungen der Formel VI, worin $X^1$ eine einfache Kovalenzbindung bezeichnet, sind bekannte oder Analoge bekannter Verbindungen. Die Verbindungen der Formel VI, worin $X^1$ eine Äthylengruppe bezeichnet, können beispielsweise dadurch hergestellt werden, dass man eine entsprechende Verbindung der Formel VI, worin $X^1$ eine einfache Kovalenzbindung bezeichnet, mit $(C_6H_5)_3P{=}CH{-}COOC_2H_5$ in Benzol zum Rückfluss erhitzt, den erhaltenen $\alpha,\beta$-ungesättigten Ester in Äthanol in Gegenwart eines Palladiumkatalysators katalytisch hydriert, den erhaltenen Ester in Diäthyläther mit Lithiumaluminiumhydrid reduziert und den erhaltenen Alkohol mit Pyridiniumchlorochromat in Methylenchlorid zum gewünschten Aldehyd oxidiert.

Die Verbindungen der Formel IV können beispielsweise dadurch hergestellt werden, dass man einen Aldehyd der Formel VI mit einem Phosphoniumsalz der Formel VII in absolutem Diäthyläther in Gegenwart von Butyllithium umsetzt und das erhaltene Dien durch Diels-Alder-Reaktion mit Tetracyanoäthylen in Diäthyläther in eine Verbindung der Formel IV überführt.

Zur Herstellung der Verbindungen der Formel V kann beispielsweise eine Verbindung der allgemeinen Formel

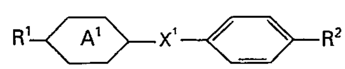

VIII

worin $R^2$ geradkettiges $C_1$-$C_{12}$-Alkoxy bezeichnet und $R^1$, $X^1$ und Ring $A^1$ die in Formel I gegebenen Bedeutungen haben, mit Lithium und flüssigem Ammoniak (vorzugsweise in einem Diäthyläther/Äthanol-Gemisch) reduziert werden. Hierbei wird im allgemeinen das 1,4-Dien oder ein Gemisch des 1,3-Diens (Verbindung der Formel V) und des 1,4-Diens erhalten. Die Isomerisierung zum 1,3-Dien kann beispielsweise mit 2,3-Dichlormaleinsäureanhydrid erfolgen. Vorzugsweise wird diese so durchgeführt, dass das in obiger Reduktion erhaltene Produkt direkt für die anschliessende Diels-Alder-Reaktion (Verfahrensvariante c) verwendet wird und 2,3-Dichlormaleinsäureanhydrid zum Reaktionsgemisch zugegeben wird. Die Verbindungen der obigen Formel VIII sind bekannte oder Analoge bekannter Verbindungen.

Die Verbindungen der obigen Formel I können in Form ihrer Gemische mit flüssigkristallinen Substanzen verwendet werden, wie z.B. mit Substanzen aus den Klassen der Schiffschen Basen, Azo- oder Azoxybenzole, Phenylbenzoate, Cyclohexancarbonsäurephenylester und -cyclohexylester, Bi- und Terphenyle, Phenylcyclohexane, Cyclohexylbiphenyle, Phenyl- und Diphenylpyrimidine, Cyclohexyl-phenylpyrimidine, Phenyldioxane und dergleichen. Derartige Substanzen sind dem Fachmann bekannt, und viele davon sind zudem im Handel erhältlich.

Die erfindungsgemässen Mischungen enthalten zweckmässigerweise etwa 1–40 Gew.-% und vorzugsweise etwa 3–25 Gew.-% an Verbindungen der Formel I.

Grundsätzlich können die Verbindungen der obigen Formel I in beliebigen flüssigkristallinen Gemischen eingesetzt werden, so z. B. auch in Gemischen mit positiver dielektrischer Anisotropie zwecks Anpassung der dielektrischen Anisotropie an die verwendete Zelle. Vorzugsweise werden die Verbindungen der obigen Formel I jedoch in Mischungen mit negativer dielektrischer Anisotropie oder in Mischungen, welche für die Zwei-Frequenz-Ansteuerung geeignet sind, verwendet. Derartige Mischungen können in an sich bekannter Weise hergestellt werden.

Vorzugsweise sind die Mischungen für die Zwei-Frequenz-Matrix-Adressierung jedoch dadurch gekennzeichnet, dass sie aus 3 Komponenten A, B und C bestehen, welche jeweils eine oder mehrere Verbindungen enthalten, und dass Komponente A eine Viskosität von höchstens 40 cp, einen Klärpunkt von mindestens 40 °C und eine dielektrische Anisotropie zwischen −2 und +1 aufweist, Komponente B eine dielektrische Anisotropie unterhalb −2 besitzt und mindestens eine Verbindung der Formel I enthält, und Komponente C eine dielektrische Anisotropie oberhalb +10, einen Klärpunkt von mindestens 100 °C und eine Cross-over-Frequenz im Gesamtgemisch von höchstens 15 kHz bei 20 °C aufweist.

Derartige Mischungen bestehen bevorzugt aus mindestens etwa 30 Gew.-% Komponente A, etwa 3–50 Gew.-% Komponente B und etwa 5–40 Gew.-% Komponente C und besonders bevorzugt aus etwa 30–87 Gew.-% Komponente A, etwa 3–40 Gew.-% Komponente B und etwa 10–30 Gew.-% Komponente C.

Verbindungen und Mischungen mit den oben, für die Komponenten A, B und C geforderten Eigenschaften sind dem Fachmann grundsätzlich bekannt. Die Gesamtmischung muss nematische oder cholesterische Eigenschaften aufweisen. Komponente A kann nematisch oder cholesterisch und Komponente C nematisch, cholesterisch oder – solange die Gesamtmischung nicht smektisch wird – auch smektisch sein. Die Komponenten A und C müssen mindestens monotrope flüssigkristalline Eigenschaften ausweisen. Bevorzugt sind jedoch diejenigen Mischungen, worin mindestens Komponente C enantiotrop flüssigkristallin ist, und besonders bevorzugt sind diejenigen Mischungen, worin Komponente A und C enantiotrop flüssigkristallin sind. Einzelne Verbindungen in den erfindungsgemässen Mischungen und Komponente B können jedoch flüssigkristallin oder nicht flüssigkristallin sein, wobei aber im letzteren Falle darauf geachtet werden sollte, dass der Mesophasenbereich der Gesamtmischung nicht zu stark eingeschränkt wird.

Verbindungen und Mischungen, die sich als Komponente A eignen, sind in grosser Zahl bekannt und vielfach auch im Handel erhältlich. Besonders geeignet sind die folgenden Verbindungen bzw. deren Mischungen:

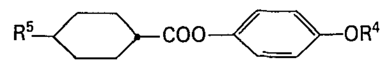 IX

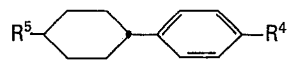 X

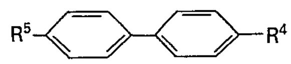 XI

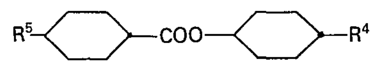 XII

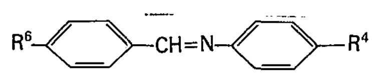 XIII

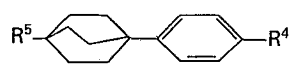 XIV

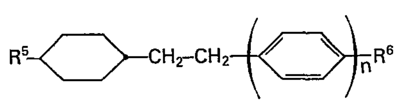 XV

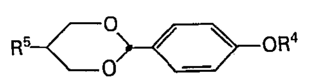 XVI

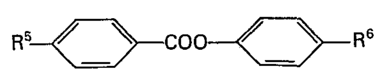 XVII

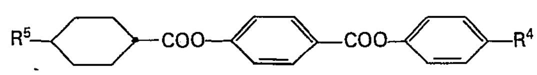 XVIII

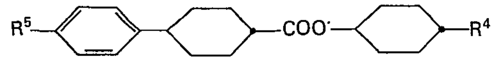 XIX

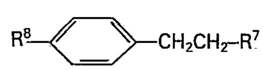 XX

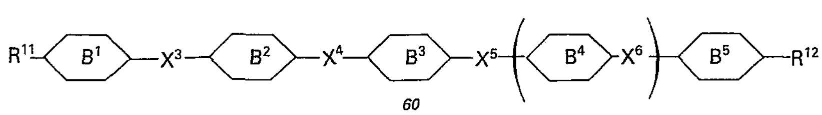 XXI

worin $R^4$ und $R^5$ geradkettige Alkylgruppen mit 1 bis 8 Kohlenstoffatomen bedeuten, $R^6$ eine geradkettige Alkyl- oder Alkoxygruppe mit 1 bis 8 Kohlenstoffatomen bezeichnet und n 1 oder 2 ist; $R^8$ trans-4-Alkylcyclohexyl, 4′-Alkyl-4-biphenylyl, p-(trans-4-Alkylcyclohexyl)phenyl, 2-(trans-4-Alkylcyclohexyl)äthyl oder p-[2-(trans-4-Alkylcyclohexyl)äthyl]phenyl und $R^7$ trans-4-Alkylcyclohexyl darstellen, oder $R^8$ trans-4-Alkylcyclohexyl und $R^7$ p-(trans-4-Alkylcycohexyl)phenyl, p-[2-(trans-4-Al-

kylcyclohexyl)äthyl]phenyl oder 4'-(trans-4-Alkylcyclohexyl)-4-biphenylyl darstellen, oder $R^8$ p-Alkylphenyl und $R^7$ p-[2-(trans-4-Alkylcyclohexyl)-äthyl]phenyl darstellen, und die Alkylreste in den Substituenten $R^7$ und $R^8$ geradkettige Gruppen mit 1 bis 7 Kohlenstoffatomen bezeichnen; m für die Zahl 0 oder 1 steht; eine der Gruppen $X^3$ oder $X^4$ eine Estergruppe -COO- oder -OOC- und die übrigen der Gruppen $X^3$, $X^4$, $X^5$ und $X^6$ eine einfache Kovalenzbindung bedeuten, oder eine dieser Gruppen auch die Äthylengruppe $-CH_2CH_2-$ bedeutet; die Ringe $B^1$ und $B^5$ eine Gruppe der Formel

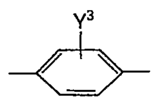

XXII

oder trans-1,4-Cyclohexylen bezeichnen; die Ringe $B^2$, $B^3$ und $B^4$ eine Gruppe der Formel XXII oder, sofern sie nicht mit mindestens einem der beiden andern dieser Ringe durch eine einfache Kovalenzbindung verknüpft sind, auch trans-1,4-Cyclohexylen darstellen; $Y^3$ Wasserstoff oder an einem der Ringe der Formel XXII, welcher nicht mit einem weiteren Ring über eine einfache Kovalenzbindung verknüpft ist, auch Fluor, Chlor oder Methyl bedeutet; und $R^{11}$ und $R^{12}$ geradkettiges Alkyl mit 1 bis 7 Kohlenstoffatomen oder an einem Ring der Formel XXII auch geradkettiges Alkoxy mit 1 bis 7 Kohlenstoffatomen bezeichnen. Besonders bevorzugt werden die Verbindungen der Formeln IX–XII, XV, XX und XXI verwendet.

Als besonders geeignete Verbindungen für Komponente B haben sich nun die Verbindungen der obigen Formel I erwiesen. Weitere, für Komponente B geeignete Verbindungen, welche im Gemisch mit einer oder mehreren Verbindungen der Formel I verwendet werden können, sind beispielsweise die in Z. Chemie 17, 333 (1977), J. prakt. Chemie 151, 221 (1938), Z. Chemie 6, 467 (1966) und Mol. Cryst. Liq. Cryst. 25, 299 (1974) erwähnten Phenyl- und Diphenylpyridazine und die in den Deutschen Offenlegungsschriften 2933563 und 2937700 beschriebenen Verbindungen mit zwei lateralen Cyanogruppen. Besonders geeignete Verbindungen, welche im Gemisch mit einer oder mehreren Verbindungen der Formel I verwendet werden können, sind jedoch die Dicyanophenylester der allgemeinen Formel

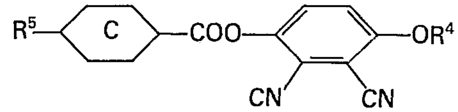

XXIII

worin $R^4$ und $R^5$ geradkettige Alkylgruppen mit 1 bis 8 Kohlenstoffatomen bedeuten und Ring C p-Phenylen oder einen trans-1,4-disubstituierten Cyclohexanring bezeichnet, und insbesondere die Cyclohexylpyridazine der allgemeinen Formel

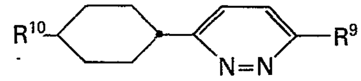

XXIV

worin $R^{10}$ eine geradkettige Alkylgruppe mit 1 bis 12 Kohlenstoffatomen bedeutet, $R^9$ eine Alkyl-, 1-Alkinyl-, Alkoxy-, p-Alkylphenyl-, p-Alkoxyphenyl- oder trans-4-Alkylcyclohexylgruppe darstellt, die Alkyl- und Alkoxyreste in $R^9$ geradkettige Reste mit 1 bis 10 Kohlenstoffatomen sind und die 1-Alkinylgruppe in $R^2$ eine geradkettige Gruppe mit 2 bis 10 Kohlenstoffatomen ist.

Unter der oben erwähnten «dielektrischen Anisotropie» der Komponente B ist im Falle nicht flüssigkristalliner Komponenten der extrapolierte Wert (aus flüssigkristallinen Mischungen, welche diese Komponente enthalten) der dielektrischen Anisotropie bei einer Temperatur, die 10 °C unter dem extrapolierten (virtuellen) Klärpunkt liegt, zu verstehen. Beispielsweise besitzen die Verbindungen der obigen Formel I dielektrische Anisotropien un etwa –15 und die Verbindungen der Formel XXIV dielektrische Anisotropien um etwa –9.

Für oder als Komponente C eignen sich beispielsweise Verbindungen mit 3 oder 4 p-Phenylen- bzw. trans-1,4-Cyclohexylengruppen, einer polaren Endgruppe und gegebenenfalls einem lateralen Halogen- oder Cyanosubstituenten. Derartige Verbindungen sind zum Teil bekannt und beispielsweise in Mol. Cryst. Liq. Cryst. 63, 129 (1981) und den Deutschen Offenlegungsschriften 2736772, 2752975 und 3046872 beschrieben.

Besonders geeignete Verbindungen für Komponente C sind die Verbindungen der allgemeinen Formel

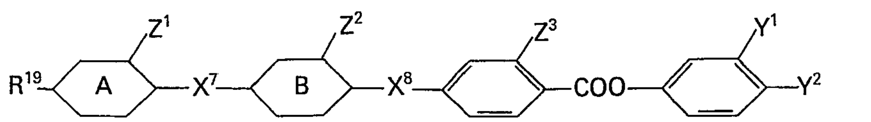

XXV

worin $X^8$ eine einfache Kovalenzbindung oder die Estergruppe -COO- bezeichnet; $X^7$ eine einfache Kovalenzbindung, die Estergruppe -COO-, die Äthylengruppe $-CH_2CH_2-$ oder, sofern $X^8$ die Estergruppe -COO- bezeichnet, auch $p-C_6H_4-$, $p-C_6H_4-CH_2CH_2-$, $-CH_2CH_2-p-C_6H_4-$, $p-C_6H_4-COO-$ oder $-COO-p-C_6H_4-$ ($p-C_6H_4-$ steht für p-Phenylen) bedeutet; Ring A für einen Benzolring oder für trans-1,4-Cyclohexylen steht; Ring B einen Benzolring oder, sofern $X^8$ die Estergruppe -COO- und $X^7$ eine einfache Kovalenzbindung, die Estergruppe -COO- oder die Äthylengruppe $-CH_2CH_2-$ bezeichnet, auch trans-1,4-Cyclohexylen darstellt; die Reste $Z^1$, $Z^2$ und $Z^3$ Wasserstoff oder an einem Benzolring, welcher nicht mit einem weiteren Ring über eine einfache Kovalenzbindung direkt verknüpft ist, auch Halogen, Cyano oder Methyl bedeuten; $Y^2$ Cyano, Nitro oder 2,2-Dicyanovinyl oder, sofern $Y^1$ für Wasserstoff steht, auch 2,2-Dicyano-1-methylvinyl darstellt; $Y^1$ Halogen, Cyano, $C_1$-$C_3$-Alkyl oder, sofern $X^7$ einen Benzolring oder eine Estergruppe aufweist oder $Y^2$ für Nitro steht oder $Z^1$ und/oder $Z^2$ von Wasserstoff verschieden sind, auch Wasserstoff bezeichnet; und $R^{19}$ $C_1$-$C_{12}$-Alkyl

oder an einem Benzolring auch $C_1$-$C_{12}$-Alkoxy bedeutet, und die Verbindungen der allgemeinen Formel

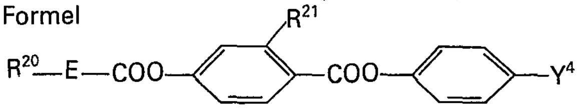

XXVI

worin $R^{21}$ Wasserstoff, Fluor, Chlor, Brom oder die Cyanogruppe bezeichnet, $Y^4$ 2,2-Dicyanovinyl, 2,2-Dicyano-1-methylvinyl oder Cyano darstellt, $R^{20}$ und E zusammen p-$R^{20}$-Phenyl, trans-4-$R^{20}$-Cyclohexyl, 4'-$R^{20}$-4-Biphenyl, p-(trans-4-$R^{20}$-Cyclohexyl)-phenyl, p-(5-$R^{20}$-2-Pyrimidinyl)phenyl, p-[2-(p'-$R^{20}$-Phenyl)äthyl]phenyl, p-[2-(trans-4-$R^{20}$-Cyclohexyl)äthyl]phenyl, trans-4-[2-(p-$R^{20}$-Phenyl)-äthyl]cyclohexyl oder trans-4-[2-(trans-4-$R^{20}$-Cyclohexyl)äthyl]-cyclohexyl bedeutet, und $R^{20}$ eine geradkettige Alkylgruppe mit 1 bis 12 Kohlenstoffatomen oder an einem Benzolring auch eine geradkettige Alkoxygruppe mit 1 bis 12 Kohlenstoffatomen bezeichnet. Diese Verbindungen weisen grosse nematische Mesophasenbereiche, niedrige Cross-over-Frequenzen und grosse absolute dielektrische Anisotropien auf. Der obige Ausdruck «Halogen» steht für Fluor, Chlor oder Brom, vorzugsweise Chlor.

Die Mischungen mit negativer dielektrischer Anisotropie enthalten zusätzlich zu einer oder mehreren Verbindungen der Formel I zweckmässigerweise eine oder mehrere weitere Verbindungen mit negativer und/oder kleiner positiver Anisotropie der Dielektrizitätskonstante (Verbindungen mit positiver dielektrischer Anisotropie dürfen bei der hier besprochenen Anwendung definitionsgemäss nur in Mengen verwendet werden, welche die Anisotropie der Gesamtmischung nicht positiv werden lassen). Das Trägermaterial (Restgemisch ohne Verbindungen der Formel I) weist zweckmässig eine dielektrische Anisotropie von höchstens etwa +1 auf. Beispiele bevorzugter Mischungskomponenten sind die oben, im Zusammenhang mit Komponenten A und B genannten Verbindungen und insbesondere die Verbindungen der Formeln IX-XII, XV, XX, XXI, XXIII und XXIV.

Die erfindungsgemässen Mischungen können ebenfalls optisch aktive Verbindungen, beispielsweise optisch aktive Biphenyle, und/oder dichroitische Farbstoffe, beispielsweise Azo-, Azoxy- oder Anthrachinonfarbstoffe, enthalten (im Falle der Mischungen für die Zwei-Frequenz-Adressierung je nach Eigenschaften als Bestandteile der Komponenten A, B und C). Der Anteil solcher Verbindungen wird durch die Löslichkeit, die gewünschte Ganghöhe (pitch), Farbe, Extinktion und dergleichen bestimmt. Vorzugsweise beträgt der Anteil optisch aktiver Verbindungen höchstens etwa 4 Gew.-% und der Anteil dichroitischer Farbstoffe höchstens etwa 10 Gewichtsprozente im Gesamtgemisch.

Die Herstellung der erfindungsgemässen flüssigkristallinen Mischungen kann in an sich bekannter Weise erfolgen, z. B. durch Erhitzen einer Mischung der Bestandteile auf eine Temperatur knapp oberhalb des Klärpunktes und anschliessendes Abkühlen.

Die Herstellung einer elektro-optischen Vorrichtung enthaltend eine erfindungsgemässe Mischung kann ebenfalls in an sich bekannter Weise erfolgen, z. B. durch Evakuieren einer geeigneten Zelle und Einbringen der Mischung in die evakuierte Zelle.

Die Verbindungen der Formel XX sind neu. Sie können anhand der folgenden Reaktionsschemata 1 und 2 hergestellt werden, worin $R^{15}$ trans-4-Alkylcyclohexyl, 4'-Alkyl-4-biphenylyl, p-(trans-4-Alkylcyclohexyl)phenyl oder 2-(trans-4-Alkylcyclohexyl)äthyl und $R^{16}$ trans-4-Alkylcyclohexyl darstellen, oder $R^{15}$ trans-4-Alkylcyclohexyl und $R^{16}$ p-(trans-4-Alkylcyclohexyl)phenyl, p-[2-(trans-4-Alkylcyclohexyl)äthyl]phenyl oder 4'-(trans-4-Alkylcyclohexyl)-4-biphenylyl darstellen, oder $R^{15}$ p-Alkylphenyl und $R^{16}$ p-[2-(trans-4-Alkylcyclohexyl)-äthyl]phenyl darstellen, und $R^{13}$ und $R^{14}$ sowie die Alkylgruppen in den Substituenten $R^{15}$ und $R^{16}$ geradkettige Alkylreste mit 1 bis 7 Kohlenstoffatomen bezeichnen.

Schema 1

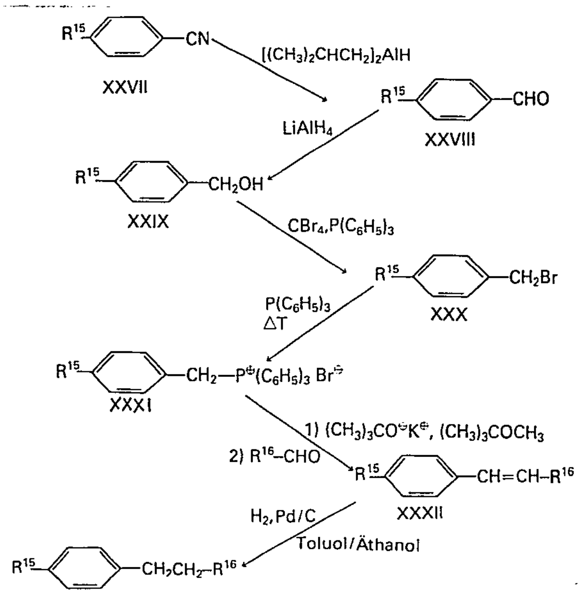


XX A

Schema 2

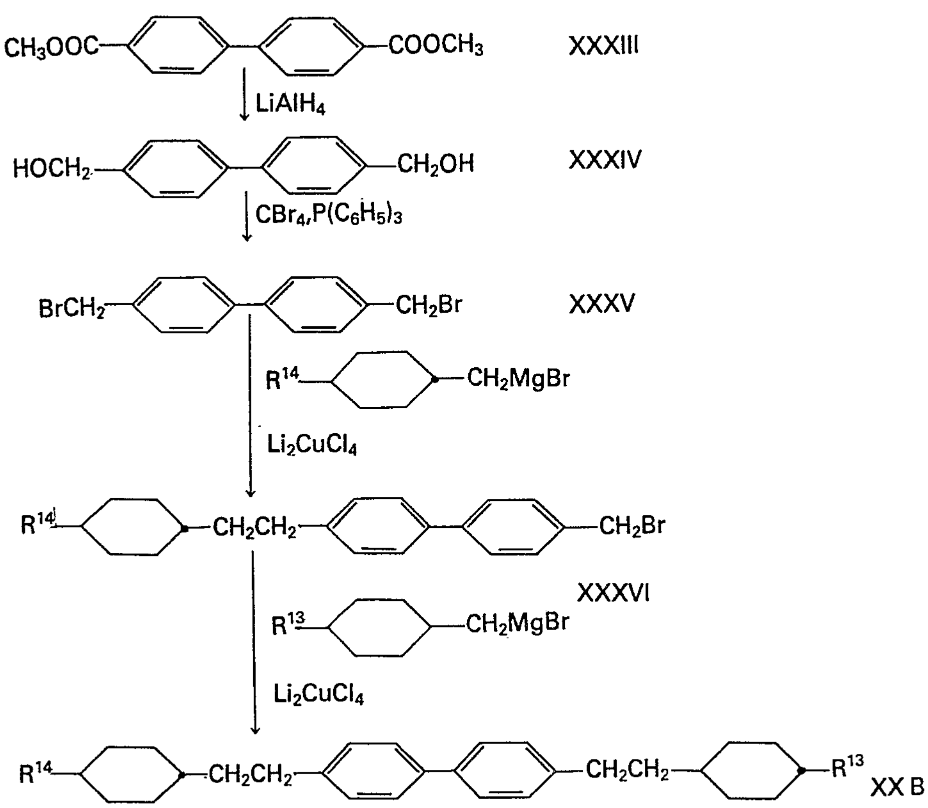


Die Verbindungen der Formel $R^{16}$ -CHO in Schema 1 können in einfacher Weise aus bekannten Verbindungen erhalten werden, z. B. die trans-4-Alkylcyclohexancarboxaldehyde durch Rosenmund-Reduktion der entsprechenden Säurechloride und die übrigen Verbindungen durch Reduktion der entsprechenden Cyanoverbindungen.

Durch Umsetzung der Verbindung der Formel XXXV mit Grignard-Reagenzien gemäss Schema 2 können Verbindungen der Formel XXXVI oder direkt Verbindungen der Formel XXB, worin $R^{13}$ und $R^{14}$ gleiche Bedeutung haben, erhalten werden. Bei der Verwendung von mindestens etwa 2 Mol Grignard-Reagens pro Mol der Verbindung der Formel XXXV wird im allgemeinen vorwiegend direkt eine Verbindung der Formel XXB gebildet.

Die Ester der Formel XXI sind ebenfalls neu. Sie können nach an sich bekannten Veresterungsmethoden (z. B. in analoger Weise zur unten beschriebenen Herstellung der Verbindungen der Formel XXV) erhalten werden. Die hierbei benötigten Ausgangsmaterialien sind bekannte oder Analoge bekannter Verbindungen und können nach bekannten Methoden hergestellt werden.

Die Verbindungen der Formel XXIV sind ebenfalls neu. Sie können in an sich bekannter Weise dadurch hergestellt werden, dass man

a) zur Herstellung der Verbindungen der Formel XXIV, worin $R^9$ eine Alkyl-, p-Alkylphenyl, p-Alkoxyphenyl oder trans-4-Alkylcyclohexylgruppe darstellt, eine Verbindung der allgemeinen Formel

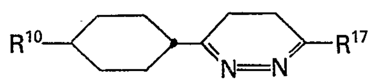

XXXVII

worin $R^{17}$ eine Alkyl-, p-Alkylphenyl, p-Alkoxyphenyl oder trans-4-Alkylcyclohexylgruppe darstellt, die Alkyl- und Alkoxyreste in $R^{17}$ geradkettige Reste mit 1 bis 10 Kohlenstoffatomen sind, und $R^{10}$ die obige Bedeutung hat, oder ein tautomeres Dihydropyridazin oxidiert (z. B. mit 2,3-Dichlor-5,6-dicyano-p-benzochinon in Dioxan, mit Natriumnitrit in Eisessig und Äthanol, mit Isopentylnitrit in Eisessig oder vorzugsweise durch katalytische Dehydrierung mit Palladium, Platin und dergleichen), oder

b) zur Herstellung der Verbindungen der Formel XXIV, worin $R^9$ eine Alkoxygruppe darstellt, eine Verbindung der allgemeinen Formel

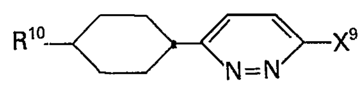

XXXVIII

worin $X^9$ Chlor oder Brom bezeichnet und $R^{10}$ die obige Bedeutung hat, mit einem Alkalimetallalkoholat (z. B. Natriummethylat) umsetzt, oder

c) zur Herstellung der Verbindungen der Formel XXIV, worin $R^9$ die Äthylengruppe darstellt, eine Verbindung der allgemeinen Formel

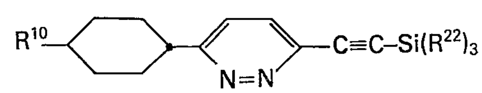

XXXIX

worin $R^{22}$ eine Alkylgruppe mit 1 bis 5 Kohlenstoffatomen bezeichnet und $R^{10}$ die obige Bedeutung hat, mit Base (z. B. Kaliumhydroxid, Natriumhydroxid oder Butyllithium) umsetzt, oder

d) zur Herstellung der Verbindungen der Formel XXIV, worin $R^9$ eine 1-Alkinylgruppe mit 3 bis 10 Kohlenstoffatomen darstellt, eine Verbindung der allgemeinen Formel

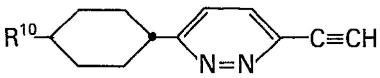

XL

worin $R^{10}$ die obige Bedeutung hat, mit Base (z.B. Butyllithium, Methyllithium, Natriumamid oder Lithiumdiisopropylamid) in das entsprechende Äthinylid überführt und mit Alkylbromid oder -jodid alkyliert.

Die Verbindungen der Formel XXXVII können durch Wanderung der Doppelbindungen im Dihydropyridazin-Ring zu tautomeren Verbindungen umlagern. Derartige Umlagerungen können z.B. durch Anwesenheit einer Spur Säure oder Base ausgelöst werden. Da die tautomeren Dihydropyridazine jedoch ebenfalls unter den obigen Bedingungen zu Verbindungen der Formel XXIV oxidiert werden können, kann gemäss Verfahrensvariante a) sowohl eine Verbindung der Formel XXXVII, als auch ein tautomeres Dihydropyridazin oder ein Gemisch solcher Verbindungen umgesetzt werden.

Die Ausgangsmaterialien der Formeln XXXVII und XXXVIII sind neu. Sie können anhand der folgenden Reaktionsschemata 3–6 hergestellt werden, worin $R^{10}$, $R^{17}$ und $X^9$ die obigen Bedeutungen haben und $R^{18}$ eine geradkettige Alkyl- oder Alkoxygruppe mit 1 bis 10 Kohlenstoffatomen darstellt.

Schema 3

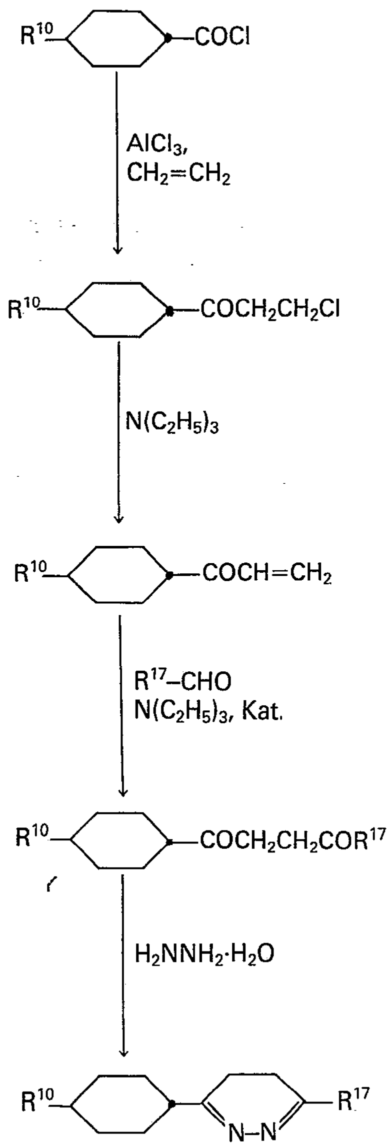


XLI

XLII

XLIII

XXXXIV

VL

XXXVII

Schema 4

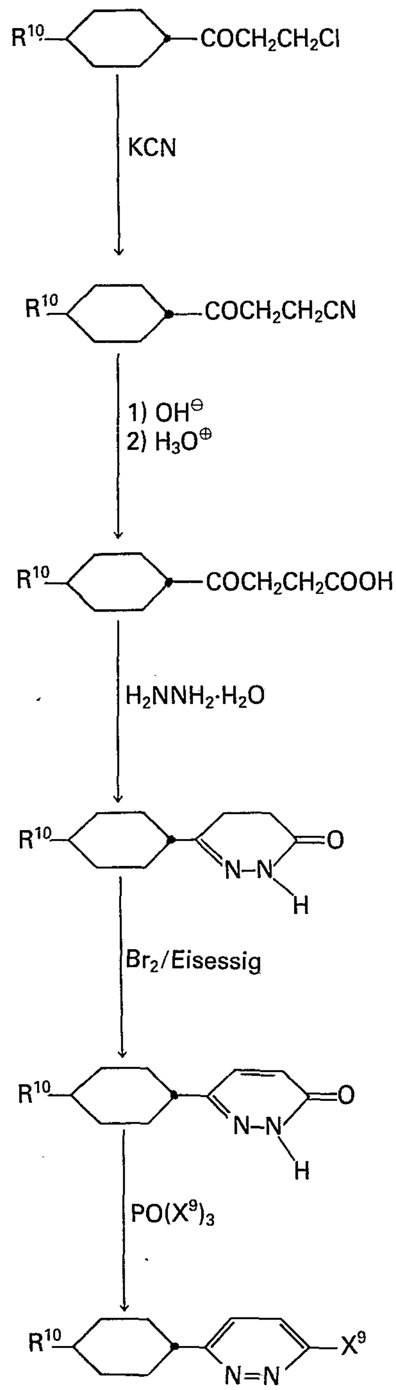


XLII

VLI

VLII

VLIII

IL

XXXVIII

Schema 5

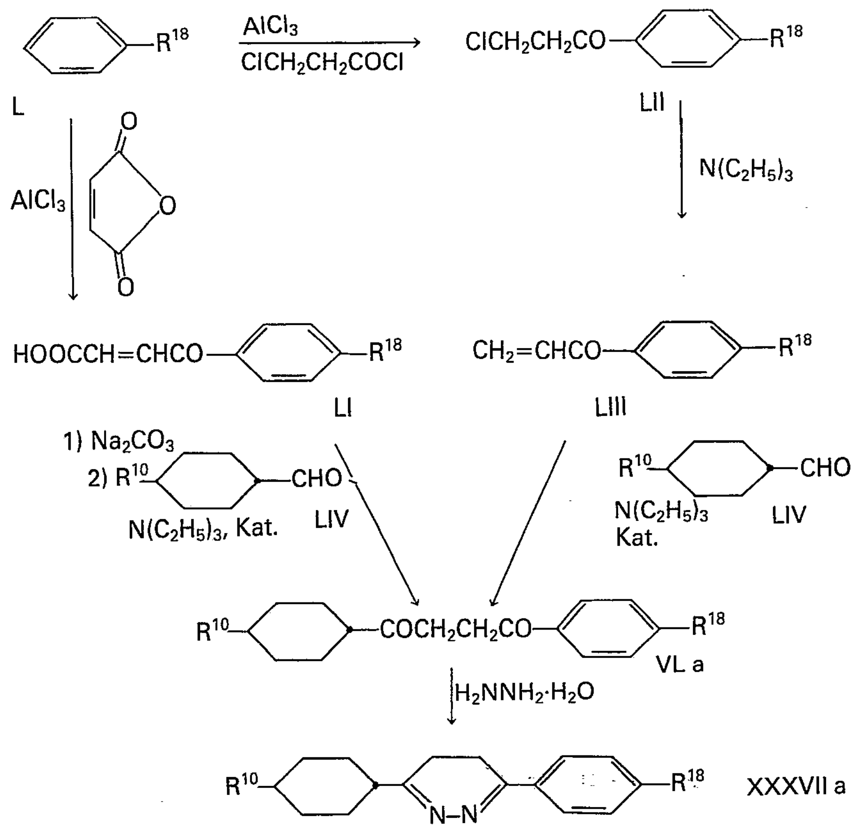


Schema 6

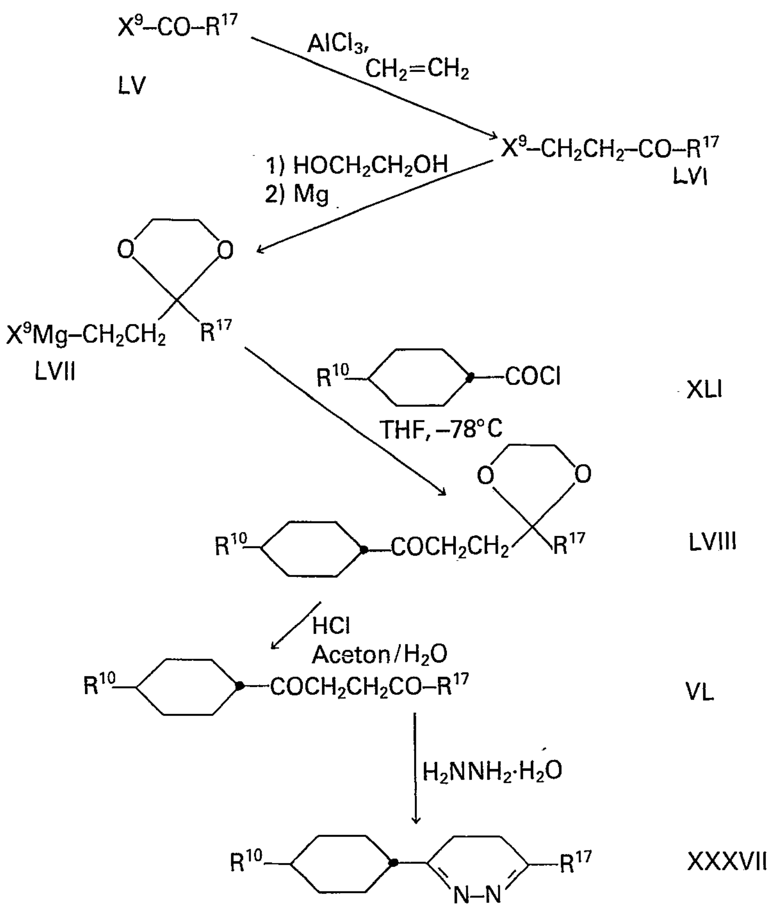

Die Ausgangssubstanzen der Formeln XLI, XXXXIV, L, LIV und LV sind bekannte oder Analoge bekannter Verbindungen und können in bekannter Weise hergestellt werden. Beispielsweise können die Aldehyde der Formel LIV durch Rosenmund-Reduktion der Säurechloride der Formel XLI hergestellt werden.

Die Addition eines Aldehydes an eine Verbindung der Formel XLIII, LI oder LIII kann sich nach der Methode von Stetter (Chem. Ber. 114 (1981) 581) in Gegenwart eines 1,3-Thiazoliumsalz-Katalysators durchgeführt werden. Bevorzugter Katalysator für die Addition eines Aldehydes der Formel LIV bzw. eines Aldehydes der Formel XXXXIV, worin $R^{17}$ Alkyl oder trans-4-Alkylcyclohexyl bedeutet, ist 3-Benzyl-5-(2-hydroxyäthyl)-4-methyl-1,3-thiazoliumchlorid und für die Addition eines Aldehydes der Formel XXXXIV, worin $R^{17}$ p-Alkylphenyl oder p-Alkoxyphenyl bedeutet, 3,4-Dimethyl-5-(2-hydroxyäthyl)-1,3-thiazoliumjodid.

Die Kopplung einer Verbindung der Formel LVII mit einer Verbindung der Formel XLI kann nach der vom T. Sato et al. in Bull. Chem. Soc. Japan 54 (1981) 505 beschriebenen Methode erfolgen.

Die Verbindungen der Formel XXXIX sind ebenfalls neu. Sie können in an sich bekannter Weise dadurch erhalten werden, dass man eine Verbindung der Formel XXXVIII in Gegenwart von Triäthylamin, Bis-(triphenylphosphin)-palladium-(II)-dichlorid und Kupfer-(I)-jodid mit Äthinyltrialkylsilan umsetzt.

Die Verbindungen der Formel XXV sind ebenfalls neu. Sie können in an sich bekannter Weise dadurch hergestellt werden, dass das Säurechlorid einer Verbindung der allgemeinen Formel

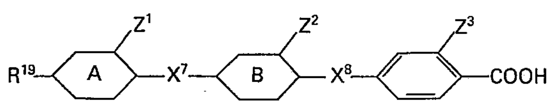

LIX

worin $R^{19}$, A, B, $X^7$, $X^8$, $Z^1$, $Z^2$ und $Z^3$ die in Formel XXV gegebenen Bedeutungen haben, mit einem Phenol der allgemeinen Formel

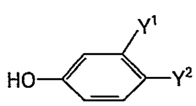

LX

worin $Y^1$ und $Y^2$ die in Formel XXV gegebene Bedeutung haben, verestert und, gewünschtenfalls, eine erhaltene Verbindung der Formel XXV, worin $Z^1$, $Z^2$ oder $Z^3$ Brom bedeutet, mit Kupfer-(I)-, Natrium- oder Kaliumcyanid umsetzt.

Die Verbindungen der Formel LX, worin $Y^2$ 2,2-Dicyanovinyl bedeutet, können beispielsweise dadurch hergestellt werden, dass man 3-$Y^1$-Anisol durch Vilsmeier-Reaktion mit Dimethylformamid in Gegenwart von Phosphoroxychlorid zu 4-Methoxy-2-$Y^1$-benzaldehyd umsetzt, dann die Methoxygruppe hydrolysiert (z.B. durch Erhitzen unter Rückfluss mit Pyridiniumchlorid und anschliessende fraktionierte Destillation) und schliesslich den erhaltenen 4-Hydroxy-2-$Y^1$-benzaldehyd durch Knoevenagel-Kondensation mit Malonitril (z.B. in Gegenwart katalytischer Mengen Eisessig und Natriumacetat in siedendem Toluol) in die Verbindung der Formel IV, worin $Y^2$ 2,2-Dicyanovinyl bedeutet, überführt. Die übrigen Verbindungen der Formel LX sind bekannte oder Analoge bekannter Verbindungen.

Die Verbindungen der Formel LIX sind ebenfalls bekannte oder Analoge bekannter Verbindungen und können nach bekannten Methoden hergestellt werden.

Die Verbindungen der Formel LIX, worin $X^8$ die Estergruppe –COO– bezeichnet, können beispielsweise dadurch hergestellt werden, dass man eine Verbindung der allgemeinen Formel

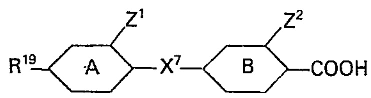

LXI

worin $X^7$, $R^{19}$, A, B, $Z^1$ und $Z^2$ die in Formel XXV gegebenen Bedeutungen haben, in Methylenchlorid in Gegenwart von Dicyclohexylcarbodiimid und 4-(Dimethylamino)pyridin mit 4-Hydroxy-2-$Z^3$-benzaldehyd verestert und den erhaltenen Aldehyd durch Jones-Oxidation mit Chromsäure und Schwefelsäure in die entsprechende Säure der Formel LIX überführt.

Bei der Herstellung der Säuren der Formel LIX, worin $X^7$ $-CH_2CH_2-$, $p-C_6H_4-CH_2CH_2-$ oder $-CH_2CH_2-p-C_6H_4-$ und $X^8$ eine einfache Kovalenzbindung bedeuten, und der Säuren der Formel LXI, worin $X^7$ $-CH_2CH_2-$, $p-C_6H_4-CH_2CH_2-$ oder $-CH_2CH_2-p-C_6H_4-$ bedeutet, erfolgt die Verknüpfung der Ringe A und B zweckmässig durch Fouquet-Schlosser-Reaktion oder durch Wittig-Reaktion. Beispielsweise kann 4-(Brommethyl)-2-$Z^2$-benzonitril, 4'-(Brommethyl)-4-biphenylcarbonitril oder trans-4-(Tosyloxymethyl)cyclohexancarbonitril in Gegenwart von Dilithiumtetrachlorkuprat mit (4-$R^{19}$-$Z^1$-Phenyl)methylmagnesiumbromid bzw. (trans-4-$R^{19}$-Cyclohexyl)methylmagnesiumbromid umgesetzt und das erhaltene Nitril zur gewünschten Säure hydrolysiert werden. Ferner kann beispielsweise 4-$R^{19}$-2-$Z^1$-Benzaldehyd oder trans-4-$R^{19}$-Cyclohexancarboxaldehyd in Gegenwart einer Base (z.B. Natriummethylat) mit (4-Methoxycarbonyl-3-$Z^2$-phenyl)methyl-triphenylphosphoniumbromid (wobei $Z^1$ und $Z^2$ Wasserstoff, Fluor, Cyano oder Methyl bedeuten) umgesetzt, dann die Doppelbindung katalytisch hydriert und schliesslich die Estergruppe verseift werden.

Die für diese Reaktionen benötigten Ausgangsmaterialien sind bekannt oder können nach an sich bekannten Methoden hergestellt werden. Beispielsweise kann 4-Alkoxy-2-$Z^1$-acetophenon durch Haloformabbau zu 4-Alkoxy-2-$Z^1$-acetophenon durch Haloformabbau zu 4-Alkoxy-2-$Z^1$-benzoesäure umgesetzt und diese durch Reduktion mit Lithiumaluminiumhydrid und Bromierung (z.B. mit Tetrabrommethan und Triphenylphosphin) in 4-Alkoxy-1-(brommethyl)-2-$Z^1$-benzol übergeführt werden. Aus 2,4-Dimethylbenzoesäure-methylester kann beispielsweise durch Umsetzung mit N-Bromsuccinimid und anschliessende Isomerentrennung 4-(Brommethyl)-2-methylben-

zoesäure-methylester erhalten werden, welcher in analoger Weise zu Org. Synth. Coll. V, 825 zu 4-Formyl-2-methylbenzoesäure-methylester umgesetzt werden kann; der nach Umsetzung mit Alkyltriphenylphosphoniumbromid und Base und anschliessender katalytischer Hydrierung der Doppelbindung erhaltene 4-Alkyl-2-methylbenzoesäure-methylester kann dann mit Natronlauge zur Säure verseift oder mit Lithiumaluminiumhydrid zum Alkohol reduziert werden, welcher schliesslich mit Bromwasserstoff zu 4-Alkyl-1-(brommethyl)-2-methylbenzol oder mit Braunstein zum 4-Alkyl-2-methylbenzaldehyd weiter umgesetzt werden kann. 1-Alkyl-3-fluorbenzol kann beispielsweise durch Umsetzung mit Butyllithium und Kohlendioxid und anschliessende Hydrolyse in 4-Alkyl-2-fluorbenzoesäure übergeführt werden und 1-Alkyl-3-chlorbenzol bzw. 1-Alkyl-3-brombenzol kann durch Friedel-Crafts-Acylierung mit Acetylchlorid in Gegenwart von Aluminiumtrichlorid und anschliessende Oxidation mit Natriumhypobromid in 4-Alkyl-2-(chlor oder brom)benzoesäure übergeführt werden; die erhaltenen Säuren können dann mit Lithiumaluminiumhydrid zum Alkohol und dieser mit Bromwasserstoff zum Bromid oder mit Braunstein zum Aldehyd weiter umgesetzt werden. Ferner kann beispielsweise 4-Methyl-2-$Z^1$-benzoesäure der Reihe nach mit Thionylchlorid, Ammoniak und Benzolsulfonylchlorid umgesetzt und das erhaltene 4-Methyl-2-$Z^1$-benzonitril mit N-Bromsuccinimid zu 4-(Brommethyl)-2-$Z^1$-benzonitril weiter umgesetzt werden.

Die Verbindungen der Formel XXVI sind zum Teil ebenfalls neue Verbindungen. Sie können in analoger Weise zu den Verbindungen der Formel XXV durch Veresterung hergestellt werden. Die hierbei benötigten Säuren können anhand des Reaktionsschemas 7 erhalten werden, worin $R^{20}$, $R^{21}$ und E die in obiger Formel XXVI gegebenen Bedeutungen haben:

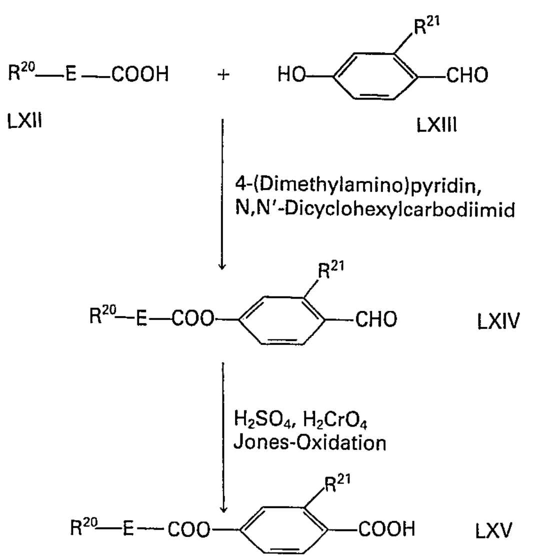

LXII
LXIII
LXIV
LXV

Die Verbindungen der Formeln LXII und LXIII sind bekannt oder können nach an sich bekannten Methoden hergestellt werden.

Die folgenden Mischungen sind Beispiele bevorzugter erfindungsgemässer Mischungen. η bedeutet die Viskosität (bulk viscosity), $f_c$ die Cross-over-Frequenz und $\Delta\varepsilon_l$ die niederfrequente («statische») und $\Delta\varepsilon_h$ die hochfrequente dielektrische Anisotropie gemessen bei 22 °C. Mischungen 1–6 besitzen negative dielektrische Anisotropie und Mischungen 7 und 8 sind für die Zwei-Frequenz-Ansteuerung geeignet.

**Mischung 1**

11,8 Gew.-% trans-4-Butylcyclohexancarbonsäure-p-äthoxyphenylester,
16,6 Gew.-% trans-4-Butylcyclohexancarbonsäure-p-pentyloxyphenylester,
10,7 Gew.-% trans-4-Pentylcyclohexancarbonsäure-p-methoxyphenylester,
12,8 Gew.-% trans-4-Pentylcyclohexancarbonsäure-p-propyloxyphenylester,
7,3 Gew.-% 4-Äthoxy-1-[2-(trans-4-pentylcyclohexyl)äthyl]benzol,
3,5 Gew.-% 3-Propyl-6-(trans-4-äthylcyclohexyl)-pyridazin,
8,3 Gew.-% 3-Propyl-6-(trans-4-pentylcyclohexyl)-pyridazin,
5,3 Gew.-% 3-Propyl-6-(trans-4-heptylcyclohexyl)-pyridazin,
10,7 Gew.-% 3-Pentyl-6-(trans-4-pentylcyclohexyl)pyridazin,
8,7 Gew.-% 4'-(trans-4-Pentylcyclohexyl)-4-[2-(trans-4-butylcyclohexyl)äthyl]biphenyl,
4,3 Gew.-% 2,3-Dicyano-1-[2-(trans-4-pentylcyclohexyl)-äthyl]-4-propylbenzol;

Smp −12 °C, Klp. 60 °C, nematisch; η = 40 cp, $\Delta\varepsilon_l = -5{,}07$

**Mischung 2**

5,6 Gew.-% trans-4-Butylcyclohexancarbonsäure-p-äthoxyphenylester,
5,1 Gew.-% trans-4-Pentylcyclohexancarbonsäure-p-methoxyphenylester,
9,8 Gew.-% trans-4-Pentylcyclohexancarbonsäure-p-propyloxyphenylester,
12,7 Gew.-% 4-Äthoxy-1-[2-(trans-4-propylcyclohexyl)-äthyl]benzol,
14,6 Gew.-% 4-Äthoxy-1-[2-(trans-4-pentylcyclohexyl)-äthyl]benzol,
10,3 Gew.-% 1-[2-(trans-4-Butylcyclohexyl)äthyl]-4-(trans-4-pentylcyclohexyl)benzol
3,4 Gew.-% 3-Propyl-6-(trans-4-äthylcyclohexyl)-pyridazin,
8,2 Gew.-% 3-Propyl-6-(trans-4-pentylcyclohexyl)-pyridazin,
5,2 Gew.-% 3-Propyl-6-(trans-4-heptylcyclohexyl)-pyridazin,
10,5 Gew.-% 3-Pentyl-6-(trans-4-pentylcyclohexyl)pyridazin,
10,3 Gew.-% 4'-(trans-4-Pentylcyclohexyl)-4-[2-(trans-4-butylcyclohexyl)äthyl]biphenyl,
4,3 Gew.-% 2,3-Dicyano-1-[2-(trans-4-pentylcyclohexyl)-äthyl]-4-propylbenzol;

Smp <0 °C, Klp. 57,8 °C, nematisch η = 31,9 cp, $\Delta\varepsilon_l = -4{,}69$

**Mischung 3**

16,5 Gew.-% trans-4-Butylcyclohexancarbonsäure-p-äthoxyphenylester,

23,3 Gew.-% trans-4-Butylcyclohexancarbonsäure-p-pentyloxyphenylester,
15,0 Gew.-% trans-4-Pentylcyclohexancarbonsäure-p-methoxyphenylester,
18,0 Gew.-% trans-4-Pentylcyclohexancarbonsäure-p-propyloxyphenylester,
10,2 Gew.-% 4-Äthoxy-1-[2-(trans-4-pentylcyclohexyl)äthyl]benzol,
7,0 Gew.-% 2,3-Dicyano-1-[2-(trans-4-pentylcyclohexyl)-äthyl]-4-propylbenzol,
10,0 Gew.-% 2,3-Dicyano-1-[2-(trans-4-pentylcyclohexyl)-äthyl]-4-butylbenzol;
Smp <0 °C, Klp. 49 °C, nematisch; $\eta$ = 45 cp, $\Delta\varepsilon_l$ = −4,01

**Mischung 4**
10,9 Gew.-% trans-4-Butylcyclohexancarbonsäure-p-äthoxyphenylester,
15,4 Gew.-% trans-4-Butylcyclohexancarbonsäure-p-pentyloxyphenylester,
9,9 Gew.-% trans-4-Pentylcyclohexancarbonsäure-p-methoxyphenylester,
11,9 Gew.-% trans-4-Pentylcyclohexancarbonsäure-p-propyloxyphenylester,
6,7 Gew.-% 4-Äthoxy-1-[2-(trans-4-pentylcyclohexyl)-äthyl]benzol,
9,6 Gew.-% 1-[2-(trans-4-Butylcyclohexyl)äthyl]-4-(trans-4-pentylcyclohexyl)benzol,
7,7 Gew.-% 4-[2-(trans-4-Butylcyclohexyl)äthyl]-4′-(trans-4-pentylcyclohexyl)-1,1′-äthylendibenzol,
3,9 Gew.-% 4-[2-(trans-4-Butylcyclohexyl)äthyl]-(trans-4-pentylcyclohexyl)biphenyl,
9,6 Gew.-% 2,3-Dicyano-1-[2-(trans-4-pentylcyclohexyl)-äthyl]-4-propylbenzol,
14,4 Gew.-% 2,3-Dicyano-1-[2-(trans-4-pentylcyclohexyl)-äthyl]-4-butylbenzol;
Smp. <0 °C, Klp. 61,5 °C, nematisch; $\eta$ = 67,9 cp, $\Delta\varepsilon_l$ = −5,22

**Mischung 5**
12,3 Gew.-% trans-4-Butylcyclohexancarbonsäure-p-äthoxy-phenylester,
17,4 Gew.-% trans-4-Butylcyclohexancarbonsäure-p-pentyloxyphenylester,
11,2 Gew.-% trans-4-Pentylcyclohexancarbonsäure-p-methoxyphenylester,
13,5 Gew.-% trans-4-Pentylcyclohexancarbonsäure-p-propyloxyphenylester,
7,6 Gew.-% 4-Äthoxy-1-[2-(trans-4-pentylcyclohexyl)-äthyl]benzol,
10,0 Gew.-% 1-[2-(trans-4-Butylcyclohexyl)äthyl]-4-(trans-4-pentylcyclohexyl)benzol,
8,0 Gew.-% 4-[2-(trans-4-Butylcyclohexyl)äthyl]-4′-(trans-4-pentylcyclohexyl)-1,1′-äthylendibenzol,
8,0 Gew.-% 2,3-Dicyano-1-[2-(trans-4-pentylcyclohexyl)-äthyl]-4-propylbenzol,
12,0 Gew.-% 2,3-Dicyano-1-[2-(trans-4-pentylcyclohexyl)-äthyl]-4-butylbenzol;
Smp. <0 °C, Klp. 65,4 °C, nematisch; $\eta$ = 47 cp, $\Delta\varepsilon_l$ = −3,71

**Mischung 6**
11,58 Gew.-% trans-4-Butylcyclohexancarbonsäure-p-äthoxyphenylester,
16,31 Gew.-% trans-4-Butylcyclohexancarbonsäure-p-pentyloxyphenylester,
10,47 Gew.-% trans-4-Pentylcyclohexancarbonsäure-p-methoxyphenylester,
12,64 Gew.-% trans-4-Pentylcyclohexancarbonsäure-p-propyloxyphenylester
7,12 Gew.-% 4-Äthoxy-1-[2-(trans-4-pentylcyclohexyl)-äthyl]benzol,
10,26 Gew.-% 4-[2-(trans-4-Butylcyclohexyl)äthyl]-4′-(trans-4-pentylcyclohexyl)biphenyl,
3,38 Gew.-% 3-Propyl-6-(trans-4-äthylcyclohexyl)-pyridazin,
8,21 Gew.-% 3-Propyl-6-(trans-4-pentylcyclohexyl)pyradizin,
10,51 Gew.-% 3-Pentyl-6-(trans-4-pentylcyclohexyl)pyridazin,
5,25 Gew.-% 3-Propyl-6-(trans-4-heptylcyclohexyl)pyridazin,
4,27 Gew.-% 2,3-Dicyano-1-[2-(trans-4-pentylcyclohexyl)äthyl]-4-propylbenzol,
Smp. <−10 °C, Klp. 61,5 °C, nematisch; $\eta$ = 42,8 cp, $\Delta\varepsilon_l$ = −5,05

**Mischung 7**
12,9 Gew.-% trans-4-Butylcyclohexancarbonsäure-p-äthoxyphenylester,
18,3 Gew.-% trans-4-Butylcyclohexancarbonsäure-p-pentyloxyphenylester,
11,7 Gew.-% trans-4-Pentylcyclohexancarbonsäure-p-methoxyphenylester,
14,1 Gew.-% trans-4-Pentylcyclohexancarbonsäure-p-propyloxyphenylester,
8,0 Gew.-% 4-Äthoxy-1-[2-(trans-4-pentylcyclohexyl)äthyl]benzol,
9,0 Gew.-% 4′-(trans-4-Pentylcyclohexyl)-4-biphenylcarbonsäure-3-chlor-4-[(3-chlor-4-nitrophenoxy)carbonyl]phenylester,
9,0 Gew.-% 4′-(trans-4-Heptylcyclohexyl)-4-biphenylcarbonsäure-3-chlor-4-[(3-chlor-4-nitrophenoxy)carbonylphenylester,
7,0 Gew.-% 2,3-Dicyano-1-[2-(trans-4-pentylcyclohexyl)-äthyl]-4-propylbenzol,
10,0 Gew.-% 2,3-Dicyano-1-[2-(trans-4-pentylcyclohexyl)-äthyl]-4-butylbenzol;
Smp. <0 °C, Klp. 76,6 °C, nematisch; $f_c$ = 440 Hz, $\Delta\varepsilon_l$ = +5,2, $\Delta\varepsilon_h$ = −5,0

**Mischung 8**
6,8 Gew.-% trans-4-Butylcyclohexancarbonsäure-p-äthoxyphenylester,
9,7 Gew.-% trans-4-Butylcyclohexancarbonsäure-p-pentyloxyphenylester,
6,3 Gew.-% trans-4-Pentylcyclohexancarbonsäure-p-methoxyphenylester,
11,2 Gew.-% trans-4-Pentylcyclohexancarbonsäure-p-propyloxyphenylester,
16,4 Gew.-% 4-Äthoxy-1-[2-(trans-4-pentylcyclohexyl)äthyl]benzol,
11,1 Gew.-% 1-[2-(trans-4-Butylcyclohexyl)äthyl]-4-(trans-4-pentylcyclohexyl)benzol,
4,5 Gew.-% 4′-Hexyl-4-biphenylcarbonsäure-3-chlor-4-[(3-chlor-4-cyanophenoxy)carbonyl]phenylester,
4,5 Gew.-% 4′-Heptyl-4-biphenylcarbonsäure-3-chlor-4-[(3-chlor-4-cyanophenoxy)carbonyl]phenylester,

4,5 Gew.-% 4'-Hexyl-4-biphenylcarbonsäure-3-chlor-4-[(3-chlor-4-nitrophenoxy)carbonyl]phenylester,

4,5 Gew.-% 4'-Heptyl-4-biphenylcarbonsäure-3-chlor-4-[(3-chlor-4-nitrophenoxy)carbonyl]phenylester,

8,2 Gew.-% 2,3-Dicyano-1-[2-(trans-4-pentylcyclohexyl)-äthyl]-4-propylbenzol,

12,3 Gew.-% 2,3-Dicyano-1-[2-(trans-4-pentylcyclohexyl)-äthyl]-4-butylbenzol;

Smp. <0 °C, Klp. 65,5 °C, nematisch; $f_c = 3{,}6$ kHz, $\Delta\varepsilon_l = +6{,}11$, $\Delta\varepsilon_h = -5{,}40$

Die Herstellung der Verbindungen der obigen Formel I wird anhand der nachstehenden Beispiele weiter veranschaulicht.

Beispiel 1

In einem Rundkolben wurde unter Stickstoff-Begasung eine Lösung (50 ml) von 19,5 g 4-Butyloxy-1-[2-(trans-4-pentylcyclohexyl)äthyl]-1,4-cyclohexadien in Tetrahydrofuran vorgelegt und unter Rühren mit einer Lösung von 50 mg 2,3-Dichlormaleinsäureanhydrid und 4,1 g Dicyanoacetylen in 50 ml Tetrahydrofuran versetzt. Das Reaktionsgemisch wurde 24 h bei Raumtemperatur gerührt und dann unter Vakuum eingedampft. Das erhaltene, dunkelbraune Öl (30,7 g) wurde an Kieselgel mit Methylenchlorid und Methylenchlorid/1% Aceton als Eluens chromatographiert. Hierbei wurden 15,7 g dunkelbraunes Öl erhalten, welches innert 1 h kontinuierlich bis auf 125 °C erwärmt wurde. Der Rückstand (15,2 g) wurde an Kieselgel mit Toluol als Eluens chromatographiert. Hierbei konnten 11,7 g brauner, fester Rückstand isoliert werden, welcher in Diäthyläther gelöst, mit Aktivkohle behandelt und filtriert wurde. Nach mehrmaliger Umkristallisation (aus Diäthyläther/Hexan, Aceton/Isopropanol und Isopropanol) und chromatographischer Aufarbeitung der Mutterlaugen wurden schliesslich 5,4 g reines 4-Butyloxy-2,3-dicyano-1-[2-(trans-4-pentylcyclohexyl)äthyl]benzol erhalten; Smp. 145,1 °C.

Das als Ausgangsmaterial verwendete 4-Butyloxy-1-[2-(trans-4-pentylcyclohexyl)äthyl]-1,4-cyclohexadien wurde wie folgt hergestellt:

In einem Sulfierkolben wurden 1306 ml flüssiges Ammoniak vorgelegt und dann unter Kühlung innert 40 min eine Lösung von 38,4 g 4-Butyloxy-1-[2-(trans-4-pentylcyclohexyl)äthyl]benzol in 1,1 l Diäthyläther zugetropft. Anschiessend wurden innert 35 min 23,3 g Lithiumdraht zugegeben und das Reaktionsgemisch 2 h gerührt. Dann wurden innert 70 min 420 ml Äthanol zum Reaktionsgemisch zugetropft und das Ammoniak über Nacht unter Stickstoff abdampfen gelassen. Das Reaktionsgemisch wurde auf 10 °C gekühlt und dann wurden 1,5 l Wasser innert 35 min so zugetropft, dass die Temperatur nicht über 10 °C stieg. Das Reaktionsgemisch wurde dreimal mit je 0,5 l Diäthyläther extrahiert. Die organischen Phasen wurden zweimal mit je 0,5 l Wasser gewaschen, über Kaliumcarbonat getrocknet, filtriert und eingeengt, wobei 39,0 g 4-Butyloxy-1-[2-(trans-4-pentylcyclohexyl)äthyl]-1,4-cyclohexadien als farbloses, kristallisierendes Öl erhalten wurden.

Beispiel 2

In einem Rundkolben mit Ölbad (120 °C) wurden 7,4 g 1,2-Dicyano-3-[2-(trans-4-pentylcyclohexyl)-äthyl]-6-propyl-1,4-cyclohexadien in 100 ml Dioxan gelöst und die Lösung mit 4,76 g 2,3-Dichloro-5,6-dicyano-p-benzochinon versetzt und während 2,5 h unter Rückfluss gekocht. Anschliessend wurde das Reaktionsgemisch auf Raumtemperatur abgekühlt und der entstandene Niederschlag abgenutscht und mit Dioxan und Diäthyläther nachgewaschen. Das Filtrat wurde am Vakuum eingeengt. Der braune, feste Rückstand (9,9 g) wurde an Kieselgel mit Toluol/Hexan (Volumenverhältnis 1:1) und Toluol als Eluens chromatographiert. Das isolierte Rohprodukt (7,0 g) wurde mehrmals umkristallisiert (aus Hexan und Isopropanol), wobei schliesslich 2,3 g reines 2,3-Dicyano-1-[2-(trans-4-pentylcyclohexyl)-äthyl]-4-propylbenzol erhalten wurden; Smp. 83,6 °C.

Das als Ausgangsmaterial verwendete 1,2-Dicyano-3-[2-(trans-4-pentylcyclohexyl)äthyl]-6-propyl-1,4-cyclohexadien wurde wie folgt hergestellt:

a) In einem Rundkolben wurden unter Stickstoff 20,4 g trans-4-Pentylcyclohexancarboxaldehyd in 500 ml Benzol gelöst und mit 49,5 g des Phosphorans $(C_6H_5)_3P{=}CHCOOC_2H_5$ [Helv. Chim. Acta 40, 1242 (1957)] versetzt. Das Gemisch wurde zum Sieden erhitzt, 6 h unter Stickstoff am Rückfluss gekocht und über Nacht stehengelassen. Nach dem Einengen am Vakuum wurden 73,2 g gelblicher, halbfester Rückstand erhalten. Chromatographie des Rückstandes an Kieselgel mit Toluol/Hexan (Volumenverhältnis 1:1) und Toluol ergab schliesslich 27,7 g β-(trans-4-Pentylcyclohexyl)-acrylsäure-äthylester als fast farbloses Öl; Sdp. 104–116 °C/0,07 mm Hg.

b) 41,6 g β-(trans-4-Pentylcyclohexyl)acrylsäure-äthylester wurden in 368 ml Äthanol gelöst, mit 7,4 g Palladium/Kohle (5%) versetzt und bei Raumtemperatur und unter Normaldruck bis zum Stillstand hydriert (Wasserstoffaufnahme 0,167 Mol). Das Reaktionsgemisch wurde mit Stickstoff begast und filtriert (Nachwaschen mit Äthanol und Methylenchlorid). Einengen des Filtrates im Vakuum ergab 38,4 g β-(trans-4-Pentylcyclohexyl)-propionsäure-äthylester als farbloses Öl, welches noch im Hochvakuum destilliert wurde; Sdp. 113–121 °C/0,05–0,06 mm Hg.

c) In einem Sulfierkolben wurden unter Stickstoffbegasung 250 ml absoluter Diäthyläther vorgelegt und vorsichtig mit 3,9 g Lithiumaluminiumhydrid versetzt. Innert 50 min wurde eine Lösung von 46,9 g β-(trans-4-Pentylcyclohexyl)propionsäure-äthylester in 200 ml absolutem Diäthyläther zugetropft. Dann wurden noch 250 ml absoluter Diäthyläther zugegeben und das Reaktionsgemisch über Nacht weitergerührt. Anschliessend wurden innert 10 min 21 ml Wasser und dann 210 ml 10%ige Schwefelsäure zum Reaktionsgemisch zugetropft. Die wässrige Phase wurde abgetrennt und zweimal mit Diäthyläther extrahiert. Die vereinigten organischen Phasen wurden je einmal mit Wasser und gesättigter Kochsalzlösung gewaschen, über Natriumsulfat getrocknet und eingedampft. Es wurden 39,2 g 3-(trans-4-Pentylcyclohexyl)-1-propanol als farbloses Öl erhalten.

d) In einem Sulfierkolben wurden unter Stickstoff-Begasung 70,2 g Pyridiniumchlorochromat in 506 ml Methylenchlorid suspendiert und dann unter gutem Rühren innert 5 min durch einen Tropftrichter mit einer Lösung von 39,2 g 3-(trans-4-Pentylcyclohexyl)-1-propanol in 60 ml Methylenchlorid versetzt. Der Tropftrichter wurde mit 20 ml Methylenchlorid gespült und dann das Reaktionsgemisch bei Raumtemperatur und unter Stickstoff-Begasung 2,5 h weitergerührt. Danach wurden 225 ml absoluter Diäthyläther zugegeben und das Reaktionsgemisch noch 15 min bei Raumtemperatur gerührt. Die überstehende Lösung wurde zum zähflüssigen, schwarzen Niederschlag abdekantiert und der Sulfierkolben viermal mit je 110 ml absolutem Diäthyläther gespült. Die vereinigten organischen Phasen wurden mit Diäthyläther als Eluens chromatographiert. Das nach dem Eindampfen im Vakuum erhaltene grünliche Öl (34,1 g) wurde im Hochvakuum unter Stickstoff destilliert, wobei 28,1 g 3-(trans-4-Pentylcyclohexyl)propionaldehyd als farbloses kristallisierendes Öl erhalten wurden (Sdp. 90–99 °C/0,08–0,1 mm Hg).

e) In einem Sulfierkolben wurden unter Stickstoffbegasung 58,2 g trans-2-Hexenyl-triphenylphosphoniumbromid in 219 ml absolutem Diäthyläther suspendiert, auf 1 °C abgekühlt und innert 25 min bei 1–4 °C durch einen Tropftrichter tropfenweise mit 89,4 ml einer 1,6M Lösung von Butyllithium in Hexan mit 41 ml absolutem Diäthyläther versetzt. Der Tropftrichter wurde mit 17 ml absolutem Diäthyläther gespült und das Reaktionsgemisch 30 min bei 0 °C weitergerührt. Anschliessend wurde innert 30 min bei 3–4 °C durch den Tropftrichter eine Lösung von 28,1 g 3-(trans-4-Pentylcyclohexyl)propionaldehyd in 68,5 ml absolutem Diäthyläther zugetropft und dann der Tropftrichter mit 17 ml absolutem Diäthyläther gespült. Die erhaltene Suspension wurde noch 2 h bei 0 °C und 1 h bei Raumtemperatur gerührt, dann mit Diäthyläther in einen Rundkolben gespült und im Vakuum eingeengt. Der Rückstand wurde mit 410 ml Methanol/Wasser (Volumenverhältnis 6:4) in einen Scheidetrichter gespült und dreimal mit Hexan extrahiert. Die Hexan-Phasen wurden einmal mit 410 ml Methanol/Wasser (Volumenverhältnis 6:4) und zweimal mit je 250 ml Wasser gewaschen, über Natriumsulfat getrocknet und eingedampft. Chromatographie des erhaltenen bräunlichen Öls (38,5 g) an Kieselgel mit Hexan als Eluens ergab schliesslich 32,7 g 1-(trans-4-Pentylcyclohexyl)-3,5-trans-nonadien (cis/trans-Verhältnis für die Doppelbindung in 3,4-Stellung 46:52,4) als farbloses Öl.

f) In einem Rundkolben wurden 9,5 g 1-(trans-4-Pentylcyclohexyl)-3,5-trans-nonadien in 62 ml Tetrahydrofuran gelöst und mit 14 mg Hydrochinon versetzt. Anschliessend wurde eine Lösung von 3,2 g Dicyanoacetylen in 18 ml Tetrahydrofuran zupipettiert und zweimal mit je 9 ml Tetrahydrofuran nachgespült. Das Reaktionsgemisch wurde 2,5 h bei Raumtemperatur und dann über Nacht bei 40–45 °C Badtemperatur unter Sickstoff gerührt. Danach wurde die dunkelbraune Reaktionslösung im Vakuum eingeengt. Das erhaltene dunkelbraune Öl (10,9 g) wurde an Kieselgel mit Hexan, Hexan/Toluol (Volumenverhältnis 1:1) und Toluol chromatographiert, wobei 4,2 g nicht umgesetztes 3-cis, 5-trans-Dien als farbloses Öl und 7,4 g 1,2-Dicyano-3-[2-(trans-4-pentylcyclohexyl)-äthyl]-6-propyl-1,4-cyclohexadien als schwach gelbliches Öl erhalten wurden.

Das oben in Absatz e) vewendete trans-2-Hexenyltriphenylphosphoniumbromid wurde wie folgt hergestellt:

g) In einem Sulfierkolben wurde in eine Lösung von 84,3 g trans-2-Hexen-1-ol in 253 ml Petroläther trockenes Kohlendioxid eingeleitet, dann die Reaktionslösung auf −15 °C abgekühlt und bei dieser Temperatur innert 1 h tropfenweise mit einer Lösung von 155,8 g Phosphortribromid in 253 ml Petroläther versetzt. Das Reaktionsgemisch wurde weitergerührt, wobei die Temperatur noch 2 h bei −15 °C gehalten und dann innert 5 h auf 0 °C und über Nacht auf Raumtemperatur steigengelassen wurde. Anschliessend wurde das Reaktionsgemisch auf 1 l Eis/Wasser gegossen. Die wässrige Phase wurde abgetrennt und dreimal mit je 250 ml Petroläther extrahiert. Die vereinigten organischen Phasen wurden der Reihe nach mit 250 ml gesättigter Kochsalzlösung, 250 ml gesättigter Natriumhydrogencarbonat-Lösung und 250 ml gesättigter Kochsalzlösung gewaschen, über Natriumsulfat getrocknet, filtriert und eingeengt. Als Rückstand wurden 134,6 g 1-Brom-2-hexen in Form eines gelblichen Öls erhalten.

h) In einem Erlenmeyer-Kolben wurden 200 g Triphenylphosphin in 1 l Benzol gelöst und mit 134,6 g 1-Brom-2-hexen versetzt. Nach einigen Minuten begann das Produkt zu kristallisieren. Das Reaktionsgemisch wurde noch 2 Tage bei Raumtemperatur stehengelassen. Anschliessend wurde der Niederschlag genutscht, mit Benzol und Petroläther gewaschen und im Vakuum über Kaliumhydroxid getrocknet. Das erhaltene Rohprodukt (292,6 g) wurde aus Äthanol umkristallisiert, mit Diäthyläther aufgekocht, genutscht, im Vakuum über Kaliumhydroxid getrocknet, dann pulverisiert und nochmals im Hochvakuum über Phosphorpentoxid getrocknet. Hierbei wurden 209,9 g trans-2-Hexenyl-triphenylphosphoniumbromid als farbloses Pulver erhalten; Smp. 146 °C. Aufarbeitung der Mutterlaugen ergab weitere 74,7 g Produkt.

In analoger Weise wurden folgende Verbindungen hergestellt:

2,3-Dicyano-4-propyl-4′-pentylbiphenyl; Smp. 61.4 °C

2′, 3′-Dicyano-4-pentyl-4″-propyl-p-terphenyl; Smp. 134,9 °C.

In analoger Weise können folgende Verbindungen hergestellt werden:
2,3-Dicyano-1-[2-(trans-4-pentylcyclohexyl)äthyl]-4-butylbenzol; Smp. 71,8 °C,
2,3-Dicyano-1-[2-(trans-4-propylcyclohexyl)äthyl]-4-hexylbenzol; Smp. 67,6 °C,
2,3-Dicyano-1-[2-(p-pentylphenyl)äthyl]-4-propylbenzol; Smp. 75,7 °C.

**Patentansprüche**

1. Verbindungen der allgemeinen Formel

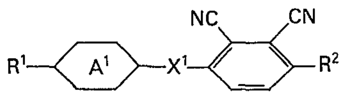
I

worin $R^1$ und $R^2$ geradkettiges $C_1$–$C_{12}$-Alkyl oder an einem aromatischen Ring auch geradkettiges $C_1$–$C_{12}$-Alkoxy bedeuten, oder einer der Reste $R^1$ und $R^2$ auch eine Gruppe der allgemeinen Formel

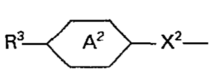
II

bedeutet; eine der Gruppen $X^1$ und $X^2$ eine einfache Kovalenzbindung und die andere eine Äthylengruppe $-CH_2CH_2-$ bezeichnet, mit der Massgabe, dass $X^1$ die Äthylengruppe $-CH_2CH_2-$ bezeichnet, wenn $R^1$ und $R^2$ geradkettiges Alkyl oder Alkoxy bedeuten; die Ringe $A^1$ und $A^2$ 1,4-Phenylen oder trans-1,4-Cyclohexylen darstellen; und $R^3$ geradkettiges $C_1$–$C_{12}$-Alkyl oder an einem aromatischen Ring $A^2$ auch geradkettiges $C_1$–$C_{12}$-Alkoxy bedeutet; mit der Massgabe, dass $R^2$ nicht für Pentyl oder Butyloxy steht, wenn $R^1$ und Ring $A^1$ zusammen 4'-Pentyl-4-biphenylyl, p-(trans-4-Pentylcyclohexyl)-phenyl oder trans-4-(trans-4-Pentylcyclohexyl)cyclohexyl bedeuten; und mit der Massgabe, dass Formel I nicht 2,3-Dicyano-1-[2-(p-pentylphenyl)äthyl]-4-(trans-4-pentylcyclohexyl)benzol bedeutet.

2. Verbindungen nach Anspruch 1, dadurch gekennzeichnet, dass $R^1$ und $R^2$ geradkettiges $C_1$–$C_{12}$-Alkyl oder an einem aromatischen Ring auch geradkettiges $C_1$–$C_{12}$-Alkoxy bedeuten.

3. Verbindungen nach Anspruch 1, dadurch gekennzeichnet, dass $X^1$ eine Äthylengruppe $-CH_2CH_2-$ und Ring $A^1$ trans-1,4-Cyclohexylen bedeuten.

4. Verbindungen nach einem der Ansprüche 1 bis 3, dadurch gekennzeichnet, dass mindestens eine der Gruppen $R^1$, $R^2$ bzw. $R^3$ geradkettiges $C_1$–$C_{12}$-Alkyl bedeutet.

5. Verbindungen nach Anspruch 4, dadurch gekennzeichnet, dass $R^1$ und $R^2$ geradkettiges $C_1$–$C_{12}$-Alkyl bedeuten oder einer dieser Reste $R^1$ und $R^2$ auch eine Gruppe der Formel II und $R^3$ geradkettiges $C_1$–$C_{12}$-Alkyl bedeuten.

6. Verbindungen nach Anspruch 5, dadurch gekennzeichnet, dass $R^1$ und $R^2$ geradkettiges $C_1$–$C_{12}$-Alkyl bedeuten.

7. Verbindungen nach Anspruch 6, dadurch gekennzeichnet, dass $R^1$ und $R^2$ geradkettiges $C_1$–$C_{12}$-Alkyl, $X^1$ eine Äthylengruppe $-CH_2CH_2-$ und Ring $A^1$ trans-1,4-Cyclohexylen bedeuten.

8. Verbindungen nach einem der Ansprüche 1 bis 7, dadurch gekennzeichnet, dass $R^1$ aund $R^2$ geradkettiges $C_3$–$C_7$-Alkyl oder an einem aromatischen Ring auch geradkettiges $C_2$–$C_6$-Alkoxy bedeuten, oder einer der Reste $R^1$ und $R^2$ auch eine Gruppe der Formel II bedeutet, und $R^3$ geradkettiges $C_3$–$C_7$-Alkyl oder an einem aromatischen Ring $A^2$ auch geradkettiges $C_2$–$C_6$-Alkoxy bezeichnet.

9. 2,3-Dicyano-1-[2-(trans-4-pentylcyclohexyl)-äthyl]-4-propylbenzol.

10. 2,3-Dicyano-1-[2-(trans-4-pentylcyclohexyl)-äthyl]-4-butylbenzol.

11. Flüssigkristallines Gemisch, bestehend aus einem flüssigkristallinen Trägermaterial mit einer dielektrischen Anisotropie von höchstens +1 und einer oder mehreren Verbindungen der allgemeinen Formel

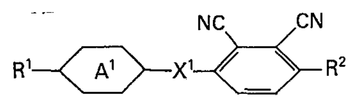
I

worin $R^1$ und $R^2$ geradkettiges $C_1$–$C_{12}$-Alkyl oder an einem aromatischen Ring auch geradkettiges $C_1$–$C_{12}$-Alkoxy bedeuten, oder einer der Reste $R^1$ und $R^2$ auch eine Gruppe der allgemeinen Formel

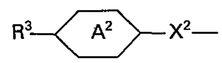
II

bedeutet; eine der Gruppen $X^1$ und $X^2$ eine einfache Kovalenzbindung und die andere eine Äthylengruppe $-CH_2CH_2-$ bezeichnet, mit der Massgabe, dass $X^1$ die Äthylengruppe $-CH_2CH_2-$ bezeichnet, wenn $R^1$ und $R^2$ geradkettiges Alkyl oder Alkoxy bedeuten; die Ringe $A^1$ und $A^2$ 1,4-Phenylen oder trans-1,4-Cyclohexylen darstellen; und $R^3$ geradkettiges $C_1$–$C_{12}$-Alkyl oder an einem aromatischen Ring $A^2$ auch geradkettiges $C_1$–$C_{12}$-Alkoxy bedeutet.

12. Flüssigkristallines Gemisch, bestehend aus einem flüssigkristallinen Trägermaterial mit einer dielektrischen Anisotropie von höchstens +1 und einer oder mehreren Verbindungen der allgemeinen Formel

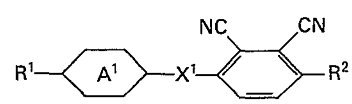
I

worin $R^1$ und $R^2$ geradkettiges $C_1$–$C_{12}$-Alkyl oder an einem aromatischen Ring auch geradkettiges $C_1$–$C_{12}$-Alkoxy bedeuten, oder einer der Reste $R^1$ und $R^2$ auch eine Gruppe der allgemeinen Formel

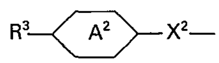
II

bedeutet; $X^1$ und $X^2$ einfache Kovalenzbindungen oder eine dieser Gruppen auch eine Äthylengruppe $-CH_2CH_2-$ bezeichnen; die Ringe $A^1$ und $A^2$ 1,4-Phenylen oder, sofern $X^1$ oder $X^2$ eine Äthylengruppe $-CH_2CH_2-$ bezeichnet, auch trans-1,4-Cyclohexylen darstellen; und $R^3$ geradkettiges $C_1$–$C_{12}$-Alkyl oder an einem aromatischen Ring $A^2$ auch geradkettiges $C_1$–$C_{12}$-Alkoxy bedeutet, dadurch gekennzeichnet, dass das Trägermaterial eine

oder mehrere Verbindungen der folgenden allgemeinen Formel enthält:

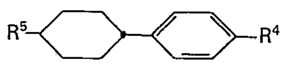
X

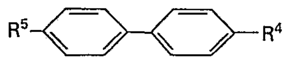
XI

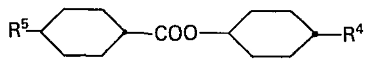
XII

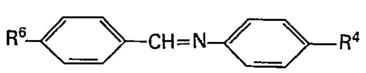
XIII

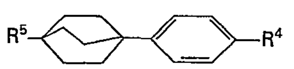
XIV

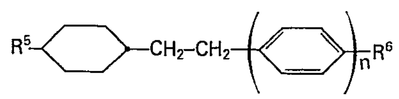
XV

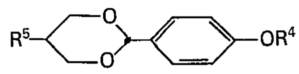
XVI

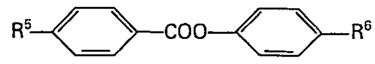
XVII

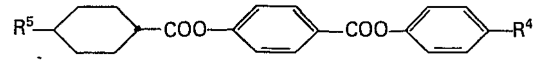
XVIII

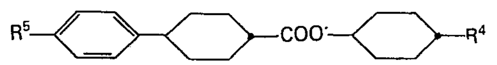
XIX

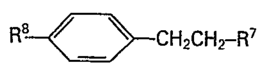
XX

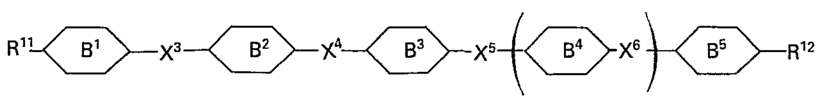
XXI

worin $R^4$ und $R^5$ gradkettige Alkylgruppen mit 1 bis 8 Kohlenstoffatomen bedeuten, $R^6$ eine geradkettige Alkyl- oder Alkoxygruppe mit 1 bis 8 Kohlenstoffatomen bezeichnet und n 1 oder 2 ist; $R^{61}$ eine geradkettige Alkylgruppe mit 1 bis 8 Kohlenstoffatomen bedeutet; $R^8$ trans-4-Alkylcyclohexyl, 4'-Alkyl-4-biphenylyl, p-(trans-4-Alkylcyclohexyl)-phenyl, 2-(trans-4-Alkylcyclohexyl)-äthyl oder p-[2-(trans-4-Alkylcyclohexyl)-äthyl]phenyl und $R^7$ trans-4-Alkylcyclohexyl darstellen, oder $R^8$ trans-4-Alkylcyclohexyl und $R^7$ p-(trans-4-Alkylcyclohexyl)phenyl, p-[2-(trans-4-Alkylcyclohexyl)äthyl]-phenyl oder 4'-(trans-4-Alkylcyclohexyl)-4-biphenylyl darstellen, oder $R^8$ p-Alkylphenyl und $R^7$ p-[2-(trans-4-Alkylcyclohexyl)äthyl]phenyl darstellen, und die Alkylreste in den Substituenten $R^7$ und $R^8$ geradkettige Gruppen mit 1 bis 7 Kohlenstoffatomen bezeichnen; m für die Zahl 0 oder 1 steht; eine der Gruppen $X^3$ oder $X^4$ eine Estergruppe -COO- oder -OOC- und die übrigen der Gruppen $X^3$, $X^4$, $X^5$ und $X^6$ eine einfache Kovalenzbindung bedeuten, oder eine dieser Gruppen auch die Äthylengruppe $-CH_2CH_2-$ bedeutet; die Ringe $B^1$ und $B^5$ eine Gruppe der Formel

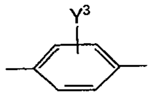
XXII

oder trans-1,4-Cyclohexylen bezeichnen; die Ringe $B^2$, $B^3$ und $B^4$ eine Gruppe der Formel XXII oder, sofern sie nicht mit mindestens einem der beiden andern dieser Ringe durch eine einfache Kovalenzbindung verknüpft sind, auch trans-1,4-Cyclohexylen darstellen; $Y^3$ Wasserstoff oder an einem der Ringe der Formel XXII, welcher nicht mit einem weiteren Ring über eine einfache Kovalenzbindung verknüpft ist, auch Fluor, Chlor- oder Methyl bedeutet; und $R^{11}$ und $R^{12}$ geradkettiges Alkyl mit 1 bis 7 Kohlenstoffatomen oder an einem Ring der Formel XXII auch geradkettiges Alkoxy mit 1 bis 7 Kohlenstoffatomen bezeichnen.

13. Flüssigkristallines Gemisch, bestehend aus 3 Komponenten A, B und C, welche jeweils eine oder mehrere Verbindungen enthalten, dadurch gekennzeichnet, dass Komponente A eine Viskosität von höchstens 40 cp, einen Klärpunkt von mindestens 40 °C und eine dielektrische Anisotropie zwischen −2 und +1 aufweist, Komponente B eine dielektrische Anisotropie unterhalb −2 besitzt und mindestens eine Verbindung der in Anspruch 11 definierten Formel I enthält, und Komponente C eine dielektrische Anisotropie oberhalb +10, einen Klärpunkt von mindestens 100 °C und eine Cross-over-Frequenz im Gesamtgemisch von höchstens 15 kHz bei 20 °C aufweist.

14. Verfahren zur Herstellung der Verbindungen der in Anspruch 11 definierten Formel I, dadurch gekennzeichnet, dass man

a) zur Herstellung der Verbindungen der Formel I, worin $R^2$ geradkettiges $C_1$–$C_{12}$-Alkyl oder eine Gruppe der Formel II bedeutet, eine Verbindung der allgemeinen Formel

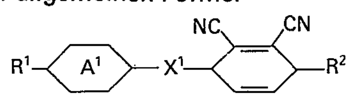
III

worin $R^2$ geradkettiges $C_1$–$C_{12}$-Alkyl oder eine Gruppe der Formel II darstellt und $R^1$, $X^1$ und Ring $A^1$ die in Anspruch 11 gegebenen Bedeutungen haben, mit 2,3-Dichloro-5,6-dicyano-p-benzochinon oder durch katalytische Dehydrierung oxydiert, oder

b) zur Herstellung der Verbindungen der Formel I, worin $R^2$ geradkettiges $C_1$–$C_{12}$-Alkyl oder eine

Gruppe der Formel II bedeutet, in einer Verbindung der allgemeinen Formel

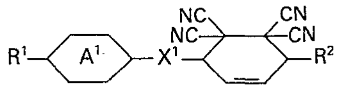

IV

worin $R^2$ geradkettiges $C_1$–$C_{12}$-Alkyl oder eine Gruppe der Formel II darstellt und $R^1$, $X^1$ und Ring $A^1$ die in Anspruch 11 gegebenen Bedeutungen haben, Cyanwasserstoff abspaltet, oder

c) Zur Herstellung der Verbindungen der Formel I, worin $R^2$ geradkettiges $C_1$–$C_{12}$-Alkoxy bedeutet, eine Verbindung der allgemeinen Formel

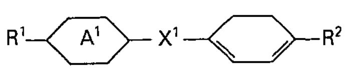

V

worin $R^2$ geradkettiges $C_1$–$C_{12}$-Alkoxy darstellt und $R^1$, $X^1$ und Ring $A^1$ die in Anspruch 11 gegebenen Bedeutungen haben, mit Dicyanoacetylen umsetzt und anschliessend Äthylen abspaltet.

15. Verwendung der Verbindungen der in Anspruch 1 definierten Formel I für elektro-optische Zwecke.

**Claims**

1. Compounds of the general formula

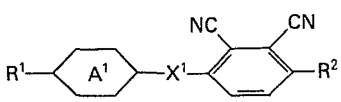

I

wherein $R^1$ and $R^2$ signify straight-chain $C_1$–$C_{12}$-alkyl or on an aromatic ring also straight-chain $C_1$–$C_{12}$-alkoxy, or one or the residues $R^1$ and $R^2$ also signifies a group of the general formula

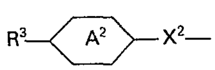

II

one of the groups $X^1$ and $X^2$ denotes a single covalent bond and the other denotes an ethylene group $-CH_2CH_2-$, with the proviso that $X^1$ denotes the ethylene group $-CH_2CH_2-$ when $R^1$ and $R^2$ signify straight-chain alkyl or alkoxy; rings $A^1$ and $A^2$ represent 1,4-phenylene or trans-1,4-cyclohexylene; and $R^3$ signifies straight-chain $C_1$–$C_{12}$-alkyl or on an aromatic ring $A^2$ also straight-chain $C_1$–$C_{12}$-alkoxy; with the proviso that $R^2$ does not stand for pentyl or butyloxy when $R^1$ and ring $A^1$ together signify 4'-pentyl-4-biphenylyl, p-(trans-4-pentylcyclohexyl)phenyl or trans-4-(trans-4-pentylcyclohexyl)cyclohexyl; and with the proviso that formula I does not signify 2,3-dicyano-1-[2-(p-pentylphenyl)ethyl]-4-(trans-4-pentylcyclohexyl)benzene.

2. Compounds according to claim 1, characterized in that $R^1$ and $R^2$ signify straight-chain $C_1$–$C_{12}$-alkyl or on an aromatic ring also straight-chain $C_1$–$C_{12}$-alkoxy.

3. Compounds according to claim 2, characterized in that $X^1$, signifies an ethylene group $-CH_2CH_2-$ and ring $A^1$ signifies trans-1,4-cyclohexylene.

4. Compounds according to any one of claims 1 to 3, characterized in that at least one of the groups $R^1$, $R^2$ and $R^3$ signifies straight-chain $C_1$–$C_{12}$-alkyl.

5. Compounds according to claim 4, characterized in that $R^1$ and $R^2$ signify straight-chain $C_1$–$C_{12}$-alkyl or one of these residues $R^1$ and $R^2$ also signifies a group of formula II and $R^3$ signifies straight-chain $C_1$–$C_{12}$-alkyl.

6. Compounds according to claim 5, characterized in that $R^1$ and $R^2$ signify straight-chain $C_1$–$C_{12}$-alkyl.

7. Compounds according to claim 6, characterized in that $R^1$ and $R^2$ signify straight-chain $C_1$–$C_{12}$-alkyl, $X^1$ signifies an ethylene group $-CH_2CH_2-$ and ring $A^1$ signifies trans-1,4-cyclohexylene.

8. Compounds according to any one of claim 1 to 7, characterized in that $R^1$ and $R^2$ signify straight-chain $C_3$–$C_7$-alkyl or on an aromatic ring also straight-chain $C_2$–$C_6$-alkoxy, or one of the residues $R^1$ and $R^2$ also signifies a group of formula II, and $R^3$ denotes straight-chain $C_3$–$C_7$-alkyl or on an aromatic ring $A^2$ also straight-chain $C_2$–$C_6$-alkoxy.

9. 2,3-Dicyano-1-[2-(trans-4-pentylcyclohexyl)-ethyl]-4-propylbenzene.

10. 2,3-Dicyano-1-[2-(trans-4-pentylcyclohexyl)-ethyl]-4-butylbenzene.

11. A liquid crystalline mixture consisting of a liquid crystalline carrier material with a dielectric anisotropy of a most +1 and one or more compounds of the general formula

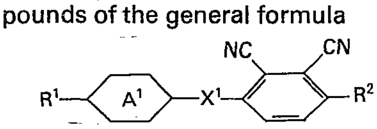

I

wherein $R^1$ and $R^2$ signify straight-chain $C_1$–$C_{12}$-alkyl or on an aromatic ring also straight-chain $C_1$–$C_{12}$-alkoxy, or one of the residues $R^1$ and $R^2$ also signifies a group of the general formula

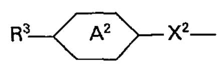

II

one of the groups $X^1$ and $X^2$ denotes a single covalent bond and the other denotes an ethylene group $-CH_2CH_2-$, with the proviso that $X^1$ denotes the ethylene group $-CH_2CH_2-$ when $R^1$ and $R^2$ signify straight-chain alkyl or alkoxy; rings $A^1$ and $A^2$ represent 1,4-phenylene or trans-1,4-cyclohexylene; and $R^3$ signifies straight-chain $C_1$–$C_{12}$-alkyl or on an aromatic ring $A^2$ also straight-chain $C_1$–$C_{12}$-alkoxy.

12. A liquid crystalline mixture consisting of a liquid crystalline carrier material with a dielectric anisotropy of at most +1 and one or more compounds of the general formula

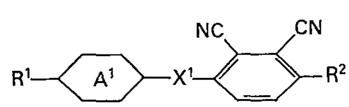

I

wherein $R^1$ and $R^2$ signify straight-chain $C_1$–$C_{12}$-alkyl or on an aromatic ring also straight-chain

$C_1$–$C_{12}$-alkoxy, or one of the residues $R^1$ and $R^2$ also signifies a group of the general formula

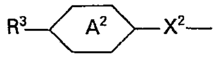
II

$X^1$ and $X^2$ denote single covalent bonds or one of these groups also denotes an ethylene group $-CH_2CH_2-$; rings $A^1$ and $A^2$ denote 1,4-phenylene or, insofar as $X^1$ or $X^2$ denotes an ethylene group $-CH_2CH_2-$, also trans-1,4-cyclohexylene; and $R^3$ signifies straight-chain $C_1$–$C_{12}$-alkyl or on an aromatic ring $A^2$ also straight-chain $C_1$–$C_{12}$-alkoxy, characterized in that the carrier material contains one or more compounds of the following general formulae:

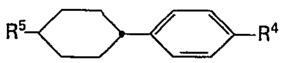
X

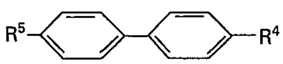
XI

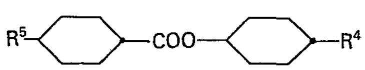
XII

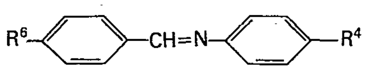
XIII

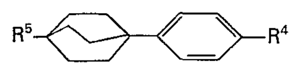
XIV

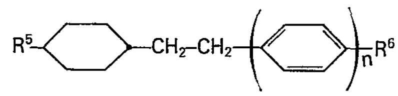
XV

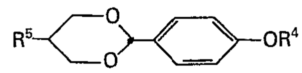
XVI

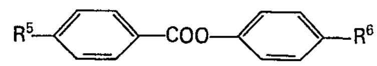
XVII

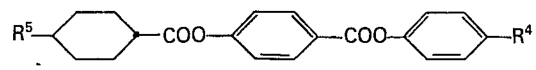
XVIII

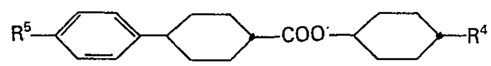
XIX

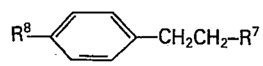
XX

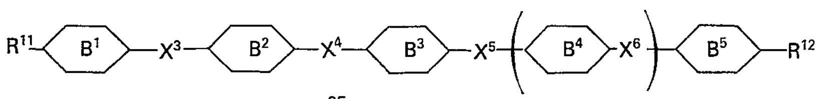
XXI

wherein $R^4$ and $R^5$ signify straight-chain alkyl groups with 1 to 8 carbon atoms, $R^6$ denotes a straight-chain alkyl or alkoxy group with 1 to 8 carbon atoms and n is 1 or 2; $R^{61}$ signifies a straight-chain alkyl group with 1 to 8 carbon atoms; $R^8$ represents trans-4-alkylcyclohexyl, 4'-alkyl-4-biphenylyl, p-(trans-4-alkylcyclohexyl)-phenyl 2-(trans-4-alkylcyclohexyl)-ethyl or p-[2-(trans-4-alkylcyclohexyl)ethyl]phenyl and $R^7$ represents trans-4-alkylcyclohexyl, or $R^8$ represents trans-4-alkylcyclohexyl and $R^7$ represents p-(trans-4-alkylcyclohexyl)phenyl, p-[2-(trans-4-alkylcyclohexyl)ethyl]phenyl or 4'-(trans-4-alkylcyclohexyl)-4-biphenylyl, or $R^8$ represents p-alkylphenyl and $R^7$ represents p-[2-(trans-4-alkylcyclohexyl)ethyl]phenyl, and the alkyl residues in the substituents $R^7$ and $R^8$ denote straight-chain groups with 1 to 7 carbon atoms; m stands for the number 0 or 1; one of the groups $X^3$ or $X^4$ signifies an ester group -COO- or -OOC- and the remainder of the groups $X^3$, $X^4$, $X^5$ and $X^6$ signify a single covalent bond, or one of these groups also signifies the ethylene group $-CH_2CH_2-$; rings $B^1$ and $B^5$ denote a group of the formula

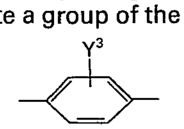
XXII

or trans-1,4-cyclohexylene; rings $B^2$, $B^3$ and $B^4$ represent a group of formula XXII or, insofar as they are not linked with at least one of the other two of these rings by a single covalent bond, also trans-1,4-cyclohexylene; $Y^3$ signifies hydrogen or on one of the rings of formula XXII, which is not linked with a further ring via a single covalent bond, also fluorine, chlorine or methyl; and $R^{11}$ and $R^{12}$ denote straight-chain alkyl with 1 to 7 carbon atoms or on a ring of formula XXII also straight-chain alkoxy with 1 to 7 carbon atoms.

13. A liquid crystalline mixture consisting of 3 components A, B and C, each of which contains one or more compounds, characterized in that component A has a viscosity of at most 40 cp, a clearing point of at least 40 °C and a dielectric anisotropy between −2 and +1, component B has a dielectric anisotropy below −2 and contains at least one compound of formula I defined in claim 11, and component C has a dielectric anisotropy above +10, a clearing point of at least 100 °C and a cross-over frequency in the total mixture of at most 15 kHz at 20 °C.

14. A process for the manufacture of the compounds of formula I defined in claim 11, characterized by

a) for the manufacture of the compounds of formula I in which $R^2$ signifies straight-chain $C_1$–$C_{12}$-alkyl or a group of formula II, oxidizing a compound of the general formula

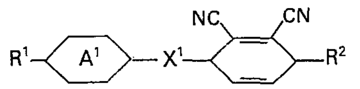

III

wherein $R^2$ represents straight-chain $C_1$–$C_{12}$-alkyl or a group of formula II and $R^1$, $X^1$ and ring $A^1$ have the significances given in claim 11, with 2,3-dichloro-5,6-dicyano-p-benzoquinone or by catalytic dehydrogenation, or

b) for the manufacture of the compounds of formula I in which $R^2$ signifies straight-chain $C_1$–$C_{12}$-alkyl or a group of formula II, cleaving off hydrogen cyanide in a compound of the general formula

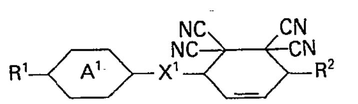

IV

wherein $R^2$ represents straight-chain $C_1$–$C_{12}$-alkyl or a group of formula II and $R^1$, $X^1$ and ring $A^1$ have the significances given in claim 11, or,

c) for the manufacture of the compounds of formula I in which $R^2$ signifies straight-chain $C_1$–$C_{12}$-alkoxy, reacting a compound of the general formula

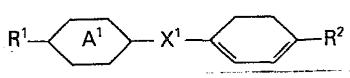

V

wherein $R^2$ represents straight-chain $C_1$–$C_{12}$-alkoxy and $R^1$, $X^1$ and ring $A^1$ have significances given in claim 11, with dicyanoacetylene and subsequently cleaving off ethylene.

15. The use of the compounds of formula I defined in claim 1 for electro-optical purposes.

**Revendications**

1. Composés de formule générale:

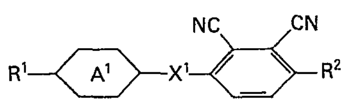

I

dans laquelle $R^1$ et $R^2$ représentent des groupes alkyle à chaîne droite en $C_1$–$C_{12}$ ou bien encore, sur un noyau aromatique, des groupes alcoxy à chaîne droite en $C_1$–$C_{12}$, ou bien encore l'un des restes $R^1$ et $R^2$ représente un groupe de formule générale:

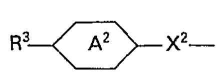

II

l'un des groupes $X^1$ et $X^2$ repésente une liaison covalente simple et l'autre un groupe éthylène $-CH_2CH_2-$, avec la condition supplémentaire que $X^1$ représente le groupe éthylène $-CH_2CH_2-$ lorsque $R^1$ et $R^2$ représentent des groupes alkyle ou alcoxy à chaîne droite; les cycles $A^1$ et $A^2$ sont le 1,4-phénylène ou le trans-1,4-cyclohexylène; et $R^3$ représente un groupe alkyle à chaîne droite en $C_1$–$C_{12}$ ou bien encore, sur un noyau aromatique $A^2$, un groupe alcoxy à chaîne droite en $C_1$–$C_{12}$; avec la condition supplémentaire que $R^2$ ne peut représenter un groupe pentyle ou butyloxy lorsque $R^1$ et le cycle $A^1$ représentent ensemble le 4'-pentyl-4-biphénylyle, le p-(trans-4-pentylcyclohexyl)-phényle ou le trans-4-(trans-4-pentylcyclohexyl)-cyclohexyle; et avec la condition supplémentaire que la formule I ne représente pas le 2,3-dicyano-1-[2-(p-pentylphényl)-éthyl]-4-(trans-4-pentylcyclohexyl)-benzène.

2. Composés selon la revendication 1, caractérisés en ce que $R^1$ et $R^2$ représentent des groupes alkyle à chaîne droite en $C_1$–$C_{12}$ ou bien encore, sur un noyau aromatique, des groupes alcoxy à chaîne droite en $C_1$–$C_{12}$.

3. Composés selon la revendication 2, caractérisés en ce que $X^1$ représente un groupe éthylène $-CH_2CH_2-$ et le cycle $A^1$ est un cycle trans-1,4-cyclohexylène.

4. Composés selon l'une des revendications 1 à 3, caractérisés en ce que l'un au moins des groupes $R^1$, $R^2$ et respectivement $R^3$ représente un groupe alkyle à chaîne droite en $C_1$–$C_{12}$.

5. Composés selon la revendication 4, caractérisés en ce que $R^1$ et $R^2$ représentent des groupes alkyle à chaîne droite en $C_1$–$C_{12}$ ou bien encore l'un de ces restes $R^1$ et $R^2$ représente un groupe de formule II et $R^3$ représente un groupe alkyle à chaîne droite en $C_1$–$C_{12}$.

6. Composés selon la revendication 5, caractérisés en ce que $R^1$ et $R^2$ représentent des groupes alkyle à chaîne droite en $C_1$–$C_{12}$.

7. Composés selon la revendication 6, caractérisés en ce que $R^1$ et $R^2$ représentent des groupes alkyle à chaîne droite en $C_1$–$C_{12}$, $X^1$ représente un groupe éthylène $-CH_2CH_2-$ et le cycle $A^1$ est le trans-1,4-cyclohexylène.

8. Composés selon l'une des revendications 1 à 7, caractérisés en ce que $R^1$ et $R^2$ représentent des groupes alkyle à chaîne droite en $C_3$–$C_7$ ou bien encore, sur un noyau aromatique, des groupes alcoxy à chaîne droite en $C_2$–$C_6$, ou bien encore l'un des restes $R^1$ et $R^2$ représente un groupe de formule II, et $R^3$ représente un groupe alkyle à chaîne droite en $C_3$–$C_7$, ou bien encore, sur un noyau aromatique $A^2$, un groupe alcoxy à chaîne droite en $C_2$–$C_6$.

9. Le 2,3-dicyano-1-[2-(trans-4-pentylcyclohexyl)-éthyl]-4-propylbenzène.

10. Le 2,3-dicyano-1-[2-(trans-4-pentylcyclohexyl)-éthyl]-4-butylbenzène.

11. Mélange à cristaux liquides consistant en un véhicule à cristaux liquides ayant une anisotropie diélectrique de +1 au maximum et un ou plusieurs composés de formule générale:

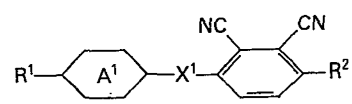

I

dans laquelle $R^1$ et $R^2$ représentent des groupes alkyle à chaîne droite en $C_1$–$C_{12}$ ou bien encore, sur un noyau aromatique, des groupes alcoxy à chaîne droite en $C_1$–$C_{12}$, ou bien l'un des restes $R^1$ et $R^2$ représente un groupe de formule générale

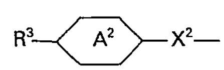

II

l'un des symboles $X^1$ et $X^2$ représente une liaison covalente simple et l'autre groupe éthylène $-CH_2CH_2-$, avec la condition supplémentaire que $X^1$ représente le groupe éthylène $-CH_2CH_2-$ lorsque $R^1$ et $R^2$ représentent des groupes alkyle ou alcoxy à chaîne droite; les cycles $A^1$ et $A^2$ sont des cycles 1,4-phénylène ou trans-1,4-cyclohexylène; et $R^3$ représente un groupe alkyle à chaîne droite en $C_1$–$C_{12}$ ou bien encore, sur un noyau aromatique $A^2$, un groupe alcoxy à chaîne droite en $C_1$–$C_{12}$.

12. Mélange à cristaux liquides consistant en un véhicule à cristaux liquides ayant une anisotropie diélectrique de +1 au maximum et un ou plusieurs composés de formule générale:

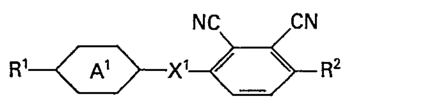
I

dans laquelle $R^1$ et $R^2$ représentent des groupes alkyle à chaîne droite en $C_1$–$C_{12}$ ou bien encore, sur un noyau aromatique, des groupes alcoxy à chaîne droite en $C_1$–$C_{12}$, ou bien encore l'un des restes $R^1$ et $R^2$ représente un groupe de formule générale:

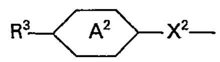
II

$X^1$ et $X^2$ représentent des liaisons covalentes simples ou bien l'un de ces groupes représente un groupe éthylène $-CH_2CH_2-$; les cycles $A^1$ et $A^2$ sont le 1,4-phénylène ou bien encore, à condition que $X^1$ ou $X^2$ représente un groupe éthylène $-CH_2CH_2-$, le trans-1,4-cyclohexylène; et $R^3$ représente un groupe alkyle à chaîne droite en $C_1$–$C_{12}$, ou bien encore, sur un noyau aromatique $A^2$, un groupe alcoxy à chaîne droite en $C_1$–$C_{12}$, caractérisé en ce que le véhicule contient un ou plusieurs composés répondant aux formules générales suivantes:

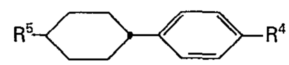
X

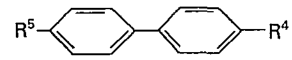
XI

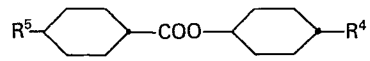
XII

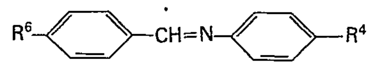
XIII

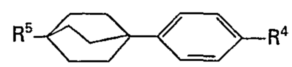
XIV

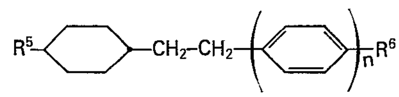
XV

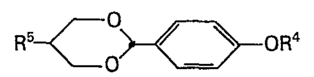
XVI

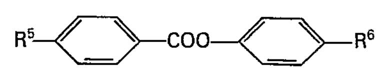
XVII

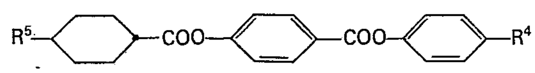
XVIII

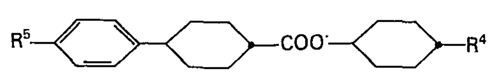
XIX

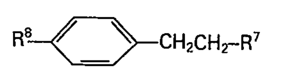
XX

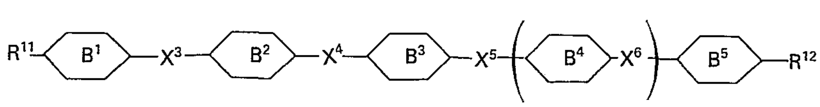
XXI

dans lesquelles $R^4$ et $R^5$ représentent des groupes alkyle à chaîne droite en $C_1$–$C_8$, $R^6$ représente un groupe alkyle ou alcoxy à chaîne droite en $C_1$–$C_8$ et n est égal à 1 ou 2; $R^{61}$ représente un groupe alkyle à chaîne droite en $C_1$–$C_8$; $R^8$ représente le trans-4-alkylcyclohexyle, 4'-alkyl-4-biphénylyle, p-(trans-4-alkylcyclohexyl)-phényle, 2-(trans-4-alkylcyclohexyl)-éthyle ou p-[2-(trans-4-alkylcyclohexyl)-éthyl]-phényle et $R^7$ représente un groupe trans-4-alkylcyclohexyle, ou bien $R^8$ représente un groupe trans-4-alkylcyclohexyle et $R^7$ un groupe p-(trans-4-alkylcyclohexyl)-phényle, p-[2-(trans-4-alkylcyclohexyl)-éthyl]-phényle ou 4'-(trans-4-alkylcyclohexyl)-4-biphénylyle, ou bien $R^8$ représente un groupe p-alkylphényle et $R^7$ un groupe p-[2-(trans-4-alkylcyclohexyl)-éthyl]-phényle, et les restes alkyle contenus dans les substituants $R^7$ et $R^8$ sont des groupes à chaîne droite en $C_1$–$C_7$; m est égal à 0 ou 1; l'un des groupes $X^3$ ou $X^4$ représente un groupe ester -COO- ou -OOC- et l'autre, ainsi que les symboles $X^5$ et $X^6$, représentent des liaisons covalentes simples, ou bien encore l'un de ces groupes représente le groupe éthylène $-CH_2CH_2-$; les cycles $B^1$ et $B^5$ sont des cycles de formule:

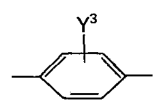
XXII

ou trans-1,4-cyclohexylène; les cycles $B^2$, $B^3$ et $B^4$ sont des cycles de formule XXII ou bien encore, à condition qu'ils ne soient pas reliés par une liaison covalente simple à l'un au moins des deux autres de ces cycles, des cycles trans-1,4-cyclohexylène, $Y^3$ représente l'hydrogène ou bien encore, sur l'un des cycles de formule XXII qui n'est pas relié à un autre cycle par une liaison covalente simple, le fluor, le chlore ou un groupe méthyle; et $R^{11}$ et $R^{12}$ représentent des groupes alkyle à chaîne droite en $C_1$–$C_7$, ou bien encore, sur un cycle de formule XXII, des groupes alcoxy à chaîne droite en $C_1$–$C_7$.

13. Mélange à cristaux liquides consistant en trois composants A, B et C contenant chacun un

ou plusieurs composés, caractérisé en ce que le composant A a une viscosité de 40 cP au maximum, un point de clarification d'au moins 40 °C et une anisotropie diélectrique allant de −2 à +1, le composant B a une anisotropie diélectrique inférieure à −2 et contient au moins un composé de formule I définie dans la revendication 11, et le composant C a une anisotropie diélectrique supérieure à +10, un point de clarification d'au moins 100 °C et un fréquence cross-over dans le mélange total de 15 kHz au maximum à 20 °C.

14. Procédé de préparation des composés de formule I définie dans la revendication 11, caractérisé en ce que

a) pour préparer les composés de formule I dans laquelle $R^2$ représente un groupe alkyle à chaîne droite en $C_1$–$C_{12}$ ou un groupe de formule II, on oxyde un composé de formule générale:

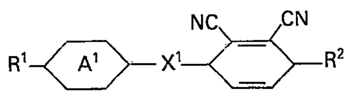 III

dans laquelle $R^2$ représente un groupe alkyle à chaîne droite en $C_1$–$C_{12}$ ou un groupe de formule II et $R^1$, $X^1$ et le cycle $A^1$ ont les significations indiquées dans la revendication 11, par la 2,3-dichloro-5,6-dicyano-p-benzoquinone ou par déshydrogénation catalytique, ou bien

b) pour préparer les composés de formule I dans laquelle $R^2$ représente un groupe alkyle à chaîne droite en $C_1$–$C_{12}$ ou un groupe de formule II, on part d'un composé de formule générale:

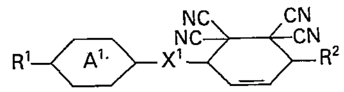 IV

dans laquelle $R^2$ représente un groupe alkyle à chaîne droite en $C_1$–$C_{12}$ ou un groupe de formule II et $R^1$, $X^1$ et le cycle $A^1$ ont les significations indiquées dans la revendication 11, et on élimine le cyanure d'hydrogène, ou bien

c) pour préparer les composés de formule I dans laquelle $R^2$ représente un groupe alcoxy à chaîne droite en $C_1$–$C_{12}$, on fait réagir un composé de formule générale:

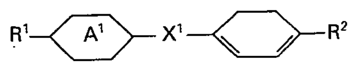 V

dans laquelle $R^2$ représente un groupe alcoxy à chaîne droite en $C_1$–$C_{12}$ et $R^1$, $X^1$ et $A^1$ ont les significations indiquées dans la revendication 11, avec le dicyano-acétylène puis on élimine de l'éthylène.

15. Utilisation des composés de formule I définie dans la revendication 1 dans des applications électro-optiques.